# EUROPEAN PATENT APPLICATION

(11) **EP 3 901 252 A1**
(43) Date of publication of application: **27.10.2021**
(21) Application number: 19898801.6
(22) Date of filing: 19.12.2019
(51) Int. Cl.: C12N 5/0735, C12N 5/09, C12N 5/10, C12N 1/00, C12Q 1/6851, C12Q 1/6876, C12N 9/99

(54) **COMPOSITION FOR REMOVING PLURIPOTENT STEM CELLS AND METHOD OF REMOVING PLURIPOTENT STEM CELLS**

(30) Priority: 21.12.2018 JP 2018239318
(71) Applicant: National University Corporation Hokkaido University, Sapporo-shi, Hokkaido 060-0808 (JP)
(72) Inventor: KONDO, Toru, Sapporo-shi, Hokkaido 060-0808 (JP)
(74) Representative: J A Kemp LLP
(86) International application number: PCT/JP2019/049837
(87) International publication number: WO 2020/130077

(57) **Abstract**

An object is to provide a composition and a method for eliminating undifferentiated pluripotent stem cells remaining in a cell group induced to differentiate from pluripotent stem cells. It has been found that while dihydroorotate dehydrogenase inhibitors exhibit cytotoxic activity against pluripotent stem cells, they do not exhibit significant cytotoxic activity against differentiated cells such as somatic stem cells.

SEQUENCE LISTING

<110>  NATIONAL UNIVERSITY CORPORATION HOKKAIDO UNIVERSITY

<120>  COMPOSITION FOR REMOVING PLURIPOTENT STEM CELLS AND METHOD OF
REMOVING PLURIPOTENT STEM CELLS

<130>  N422320EP

<150>  JP2018-239318
<151>  2018-12-21

<160>  13

<170>  PatentIn version 3.5

<210>  1
<211>  22
<212>  DNA
<213>  Artificial Sequence

<220>
<223>  Artificially synthesized primer sequence

<400>  1
tgaagaagga taagtacacg ct                                                22


<210>  2
<211>  21
<212>  DNA
<213>  Artificial Sequence

<220>
<223>  Artificially synthesized primer sequence

<400>  2
tcctgcatca tgctgtagct g                                                 21


<210>  3
<211>  21
<212>  DNA
<213>  Artificial Sequence

<220>
<223>  Artificially synthesized primer sequence

<400>  3
ctgaagcaga agaggatcac c                                                 21


<210>  4
<211>  21
<212>  DNA
<213>  Artificial Sequence

<220>
<223>  Artificially synthesized primer sequence

<400>  4
ccgcagctta cacatgttct t                                                 21

<210>  5
<211>  22
<212>  DNA
<213>  Artificial Sequence

<220>
<223>  Artificially synthesized primer sequence

<400>  5
ctgattcttc taccagtccc aa                                                22


<210>  6
<211>  21
<212>  DNA
<213>  Artificial Sequence

<220>
<223>  Artificially synthesized primer sequence

<400>  6
agagttcttg catctgctgg a                                                 21


<210>  7
<211>  20
<212>  DNA
<213>  Artificial Sequence

<220>
<223>  Artificially synthesized primer sequence

<400>  7
cggacaggat tgacagattg                                                   20


<210>  8
<211>  20
<212>  DNA
<213>  Artificial Sequence

<220>
<223>  Artificially synthesized primer sequence

<400>  8
caaatcgctc caccaactaa                                                   20


<210>  9
<211>  19
<212>  DNA
<213>  Artificial Sequence

<220>
<223>  Artificially synthesized primer sequence

<400>  9
agaattcaat gggggaaca                                                    19


<210>  10
<211>  17

<212>  DNA
<213>  Artificial Sequence

<220>
<223>  Artificially synthesized primer sequence

<400>  10
ttggattctc tgcggtc                                                      17


<210>  11
<211>  18
<212>  DNA
<213>  Artificial Sequence

<220>
<223>  Target sequence of shRNA

<400>  11
ggctagctgt ctctctct                                                     18


<210>  12
<211>  18
<212>  DNA
<213>  Artificial Sequence

<220>
<223>  Target sequence of shRNA

<400>  12
ggaagctgtg tctctcta                                                     18


<210>  13
<211>  19
<212>  DNA
<213>  Artificial Sequence

<220>
<223>  Target sequence of shRNA

<400>  13
gcaagctgac ccgtgttca                                                    19

## Description

### [Technical Field]

The present invention relates to a composition for eliminating pluripotent stem cells and a method for eliminating pluripotent stem cells. More specifically, the present invention relates to a composition and a method for eliminating undifferentiated pluripotent stem cells remaining in a cell group induced to differentiate from pluripotent stem cells.

### [Background Art]

Pluripotent stem cells, such as embryonic stem cells (ES cells) and induced pluripotent stem cells (iPS cells), are expected to play a key role in the realization of regenerative medicine because they have the ability to differentiate into all cells that make up the living organism. To date, a number of methods have been established to induce the differentiation of these pluripotent stem cells into specific functional cells for transplantation that are required for therapy.

However, undifferentiated pluripotent stem cells remaining after differentiation induction may form tumors. This is therefore a major obstacle to proceeding with transplantation therapy, and various methods to eliminate pluripotent stem cells have been developed to solve this problem (NPLs 1 to 5).

However, due to insufficient verification of the safety of these methods on other cells, a method to eliminate pluripotent stem cells with fewer side effects has not yet been put into practical use.

### [Citation List]

### [Non Patent Literature]

[NPL 1] Tang et al., Nat Biotech, 2011, Volume 29, pp. 829 to 834
[NPL 2] Ben-David et al., Cell Stem Cell, 2013, Volume 12, pp. 167 to 179
[NPL 3] Shiraki et al., Cell Metab, 2014, Volume 19, pp. 780-794
[NPL 4] Parr et al., Sci Rep, September 9, 2016; 6: 32532
[NPL 5] Kuang et al., Cell Chem Biol, 2017, Volume 24, pp. 685-694
[NPL 6] Sykes et al., Cell, 2016, Volume 167, pp. 171 to 186
[NPL 7] Ladds et al., Nat Commun, March 16, 2018, 9(1): 1107

### [Summary of Invention]

### [Technical Problem]

The present invention has been made in view of the problems of the aforementioned related art, and aims to find a compound that causes cytotoxicity to undifferentiated pluripotent stem cells while being less toxic to other cells. Furthermore, it is an object of the present invention to provide a composition for eliminating pluripotent stem cells containing the compound as an active ingredient, and a method for eliminating pluripotent stem cells using the compound.

### [Solution to Problem]

The present inventor has made earnest studies in order to solve the above problems, and have found as a result that a dihydroorotate dehydrogenase (DHODH) inhibitor exhibits cytotoxic activity against pluripotent stem cells such as ES cells and iPS cells. Also, the present inventor has revealed that the DHODH inhibitor does not exhibit significant cytotoxicity to differentiated cells (somatic cells such as astrocytes, and somatic stem cells such as neural stem cells).

Furthermore, the present inventor has confirmed that the transplantation of pluripotent stem cells into mice after treatment with a DHODH inhibitor or the administration of a DHODH inhibitor to mice transplanted with pluripotent stem cells can inhibit tumor formation from pluripotent stem cells without causing any particular side effects in the mice.

DHODH is an oxidoreductase that catalyzes the fourth chemical reaction of the de novo synthesis of pyrimidines. It has already been shown that DHODH inhibitors inhibit the proliferation of T cells and B cells by inhibiting their synthesis, thereby exerting an immunosuppressive effect, and because of this effect, such drugs are used for the treatment of autoimmune diseases such as rheumatoid arthritis. Furthermore, it has been reported that DHODH inhibitors have therapeutic effects on leukemia (acute myelogenous leukemia and chronic myelogenous leukemia) by removing the inhibition of differentiation in cancer stem cells or by enhancing the synthesis of p53 in those cells (NPLs 6 and 7).

However, although the effect of DHODH inhibitors on pluripotent stem cells has not been verified or suggested in any way, the present inventor is the first to show that pluripotent stem cells could be eliminated by such drugs, as described above. Meanwhile, the present inventor has found that DHODH inhibitors do not show significant cytotoxicity against somatic stem cells (such as neural stem cells), which are also classified as stem cells and have multipotency and self-renewal, albeit less so than pluripotent stem cells. Thus, the present invention has been completed.

Specifically, the present invention relates to a composition for eliminating pluripotent stem cells and a method for eliminating pluripotent stem cells using a DHODH inhibitor, and more specifically provides the following.
<1> A composition for eliminating undifferentiated pluripotent stem cells remaining in a cell group induced to differentiate from pluripotent stem cells, comprising: a dihydroorotate dehydrogenase inhibitor as an active ingredient.
<2> The composition according to <1>, wherein the pluripotent stem cells are at least one pluripotent stem cell selected from the group consisting of embryonic stem cells (ES cells), induced pluripotent stem cells (iPS cells), and embryonal carcinoma cells (EC cells).
<3> The composition according to <1> or <2>, wherein the dihydroorotate dehydrogenase inhibitor is brequinar.
<4> The composition according to any one of <1> to <3>, wherein the cell group is a cell group containing somatic stem cells induced to differentiate from the pluripotent stem cells.
<5> A method for eliminating undifferentiated pluripotent stem cells remaining in a cell group, comprising: contacting a cell group induced to differentiate from pluripotent stem cells with a dihydroorotate dehydrogenase inhibitor.
<6> The method according to <5>, wherein the pluripotent stem cells are at least one pluripotent stem cell selected from the group consisting of embryonic stem cells (ES cells), induced pluripotent stem cells (iPS cells), and embryonal carcinoma cells (EC cells).
<7> The method according to <5> or <6>, wherein the dihydroorotate dehydrogenase inhibitor is brequinar.
<8> The method according to any one of <5> to <7>, wherein the cell group is a cell group containing somatic stem cells induced to differentiate from the pluripotent stem cells.

### [Advantageous Effects of Invention]

According to the present invention, it is possible to eliminate undifferentiated pluripotent stem cells without causing significant cytotoxicity to the differentiated cells. That is, according to the present invention, it is possible to eliminate undifferentiated pluripotent stem cells remaining in a cell group induced to differentiate from pluripotent stem cells.

### [Brief Description of Drawings]

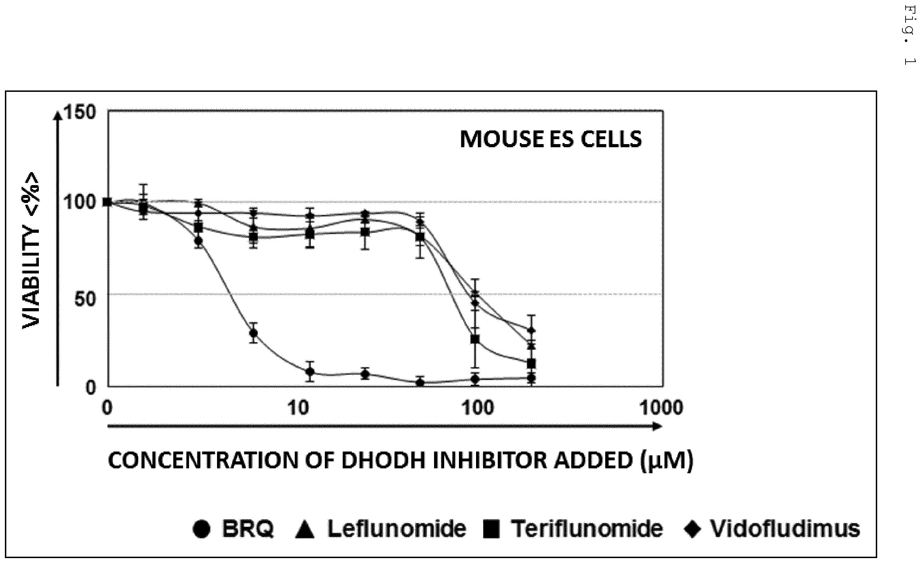

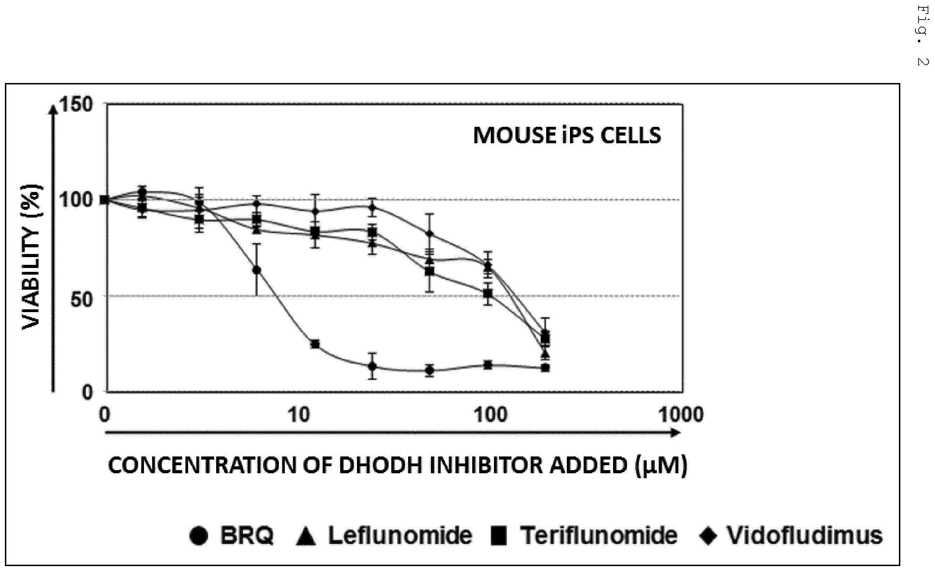

[Fig. 1] Fig. 1 is a graph showing the viability of mouse embryonic stem (ES) cells after culturing for 3 days in the presence of dihydroorotate dehydrogenase (DHODH) inhibitors. In the figure, "BRQ," "Leflunomide," "Teriflunomide," and "Vidofludimus" indicate the viability of cells in the presence of the respective DHODH inhibitors (brequinar, leflunomide, teriflunomide, and vidofludimus) (the notation in the figure is the same as in Fig. 2).
[Fig. 2] Fig. 2 is a graph showing the viability of mouse induced pluripotent stem (iPS) cells after culturing for 3 days in the presence of DHODH inhibitors.

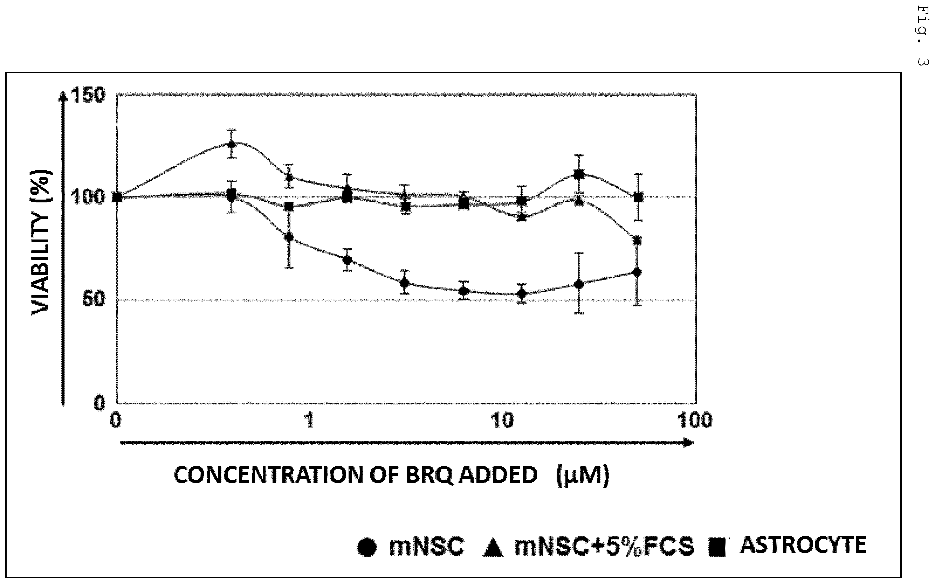

[Fig. 3] Fig. 3 is a graph showing the viability of mouse neural stem cells and the like after culturing for 3 days in the presence of BRQ. In the figure, "mNSC," "mNSC+5%FCS," and "Astrocyte" indicate the viability of mouse neural stem cells, mouse neural stem cells with 5% fetal bovine serum, and mouse astrocytes, respectively, in the presence of BRQ.

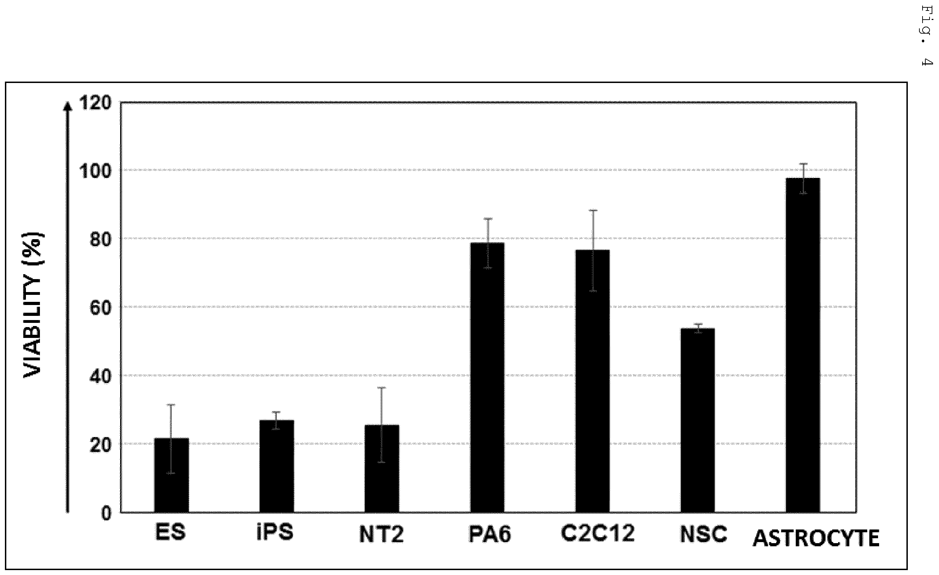

[Fig. 4] Fig. 4 is a graph showing the viability of various cells after culturing for 3 days in the presence of 10 µM BRQ. In the figure, "ES," "iPS," "NT2, " "PA6," "C2C12," "NSC," and "Astrocyte" indicate the viability of mouse ES cells, mouse iPS cells, human embryonal carcinoma cells, mouse bone marrow-derived stromal cells, mouse myoblasts, mouse neural stem cells, and mouse astrocytes, respectively.

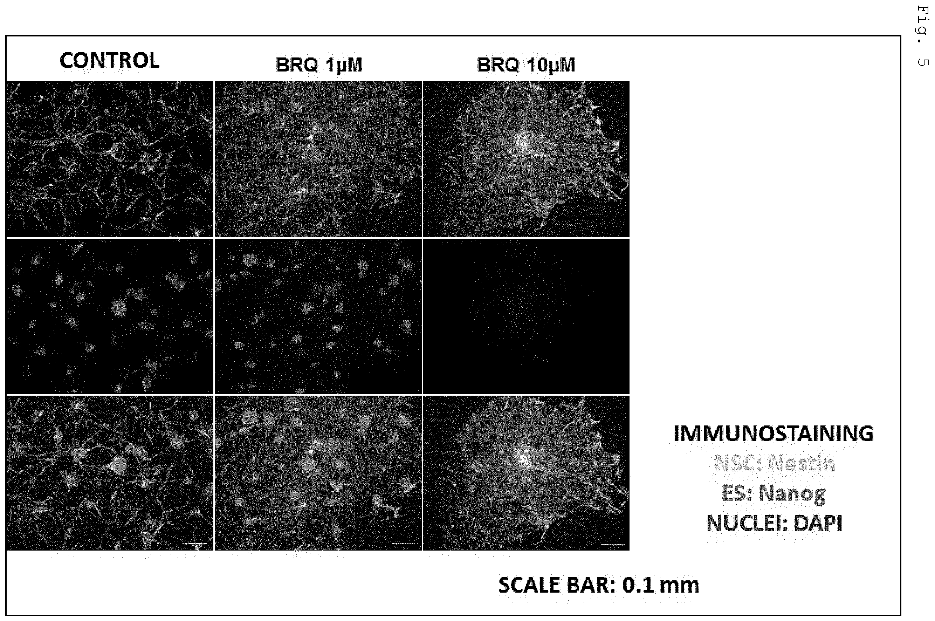

[Fig. 5] Fig. 5 provides fluorescence micrographs showing the results of mixed culture of mouse neural stem cells and mouse ES cells for 3 days in the presence of BRQ. In the figure, "Control" indicates the culture results in the absence of BRQ. In the figure, the three photographs in the upper row show the results of immunostaining for a marker of neural stem cells (Nestin). The three photographs in the middle row show the results of immunostaining for a marker of pluripotent stem cells (Nanog). The three photographs in the lower row show the results of Nestin and Nanog immunostaining, superimposed on the results of contrast staining of cell nuclei with DAPI.

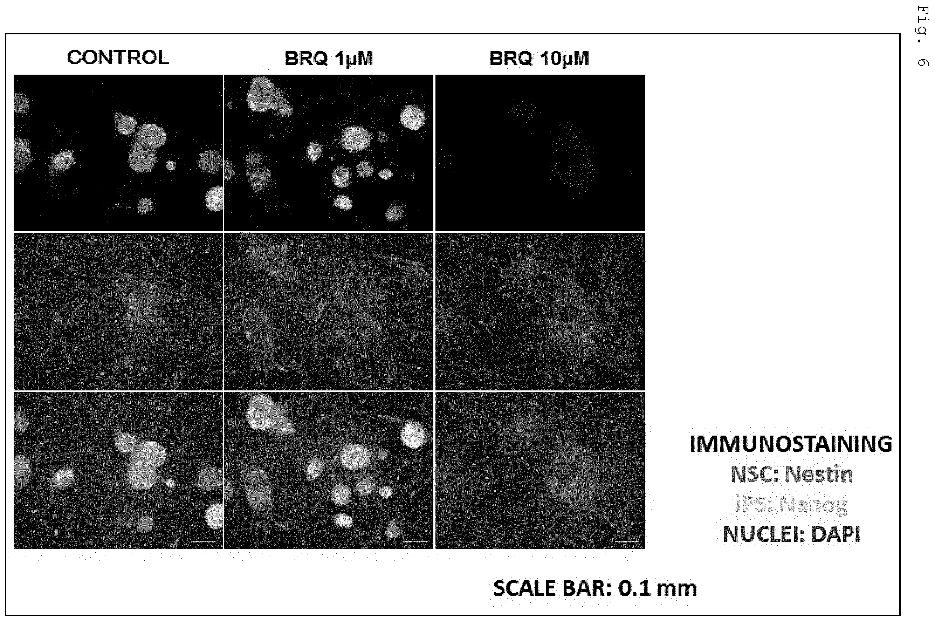

[Fig. 6] Fig. 6 provides fluorescence micrographs showing the results of mixed culture of mouse neural stem cells and mouse iPS cells for 3 days in the presence of BRQ. In the figure, "Control" indicates the culture results in the absence of BRQ. In the figure, the three photographs in the upper row show the results of immunostaining for a marker of pluripotent stem cells (Nanog). The three photographs in the middle row show the results of immunostaining for a marker of neural stem cells (Nestin). The three photographs in the lower row show the results of immunostaining for Nestin and Nanog, superimposed on the results of contrast staining of cell nuclei with DAPI.

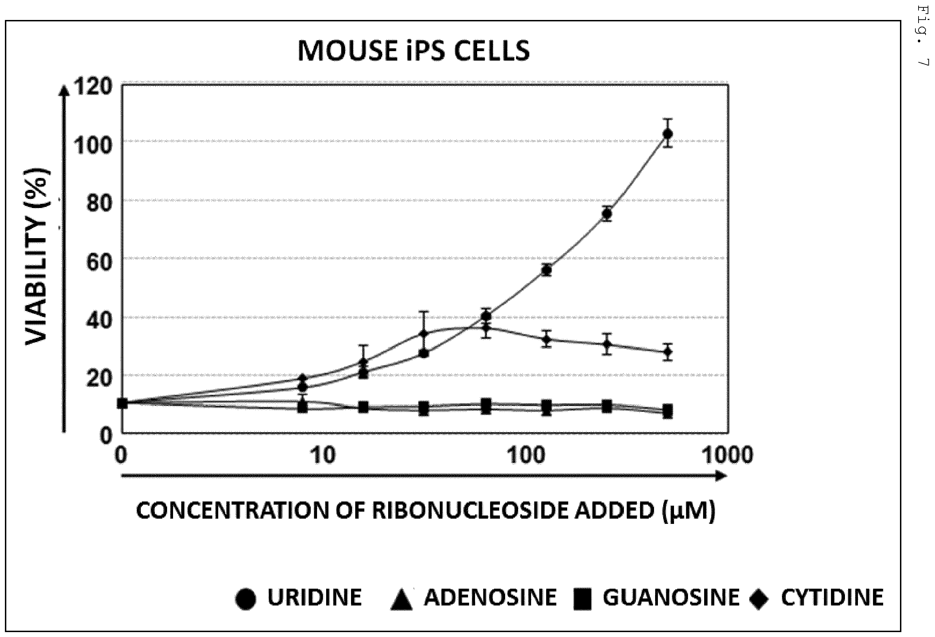

[Fig. 7] Fig. 7 is a graph showing the viability of mouse iPS cells when various ribonucleosides (uridine, adenosine, guanosine, or cytidine) are added in the presence of 10 µM BRQ.

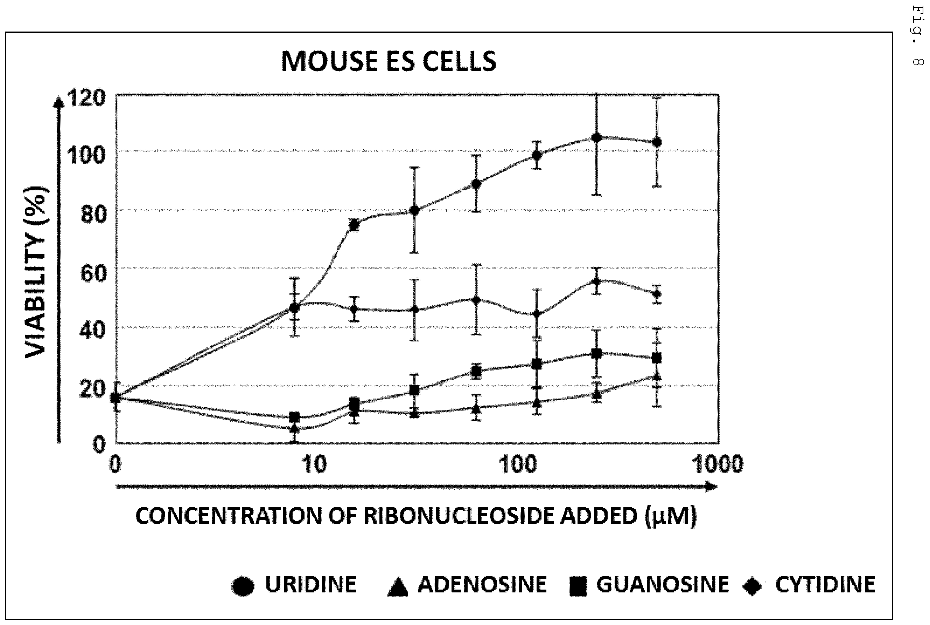

[Fig. 8] Fig. 8 is a graph showing the viability of mouse ES cells when various ribonucleosides (uridine, adenosine, guanosine, or cytidine) are added in the presence of 10 µM BRQ.

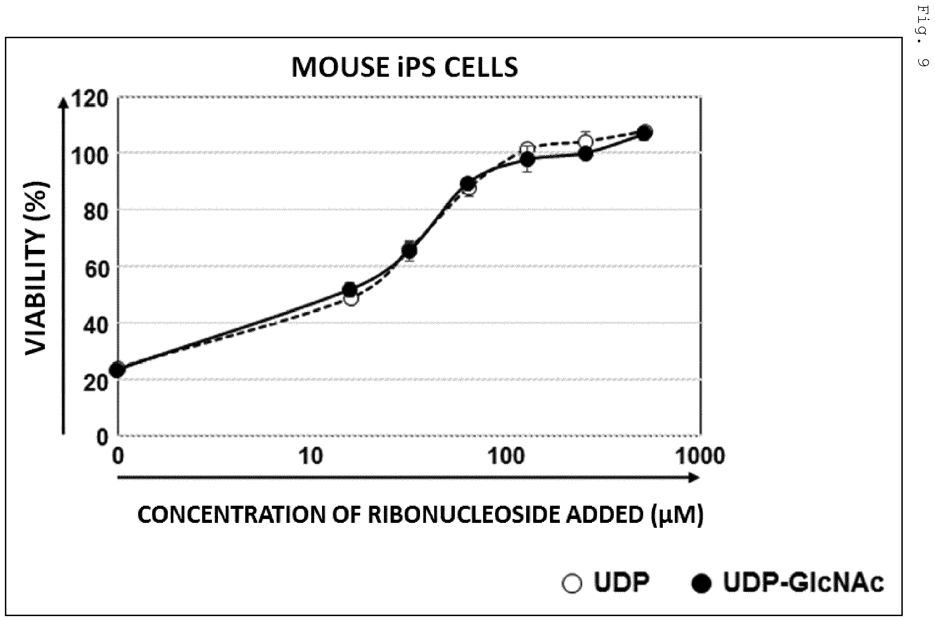

[Fig. 9] Fig. 9 shows the viability of mouse iPS cells when various ribonucleosides (uridine diphosphate (UDP) or uridine diphosphate-N-acetylglucosamine (UDP-GlcNAc)) are added in the presence of 10 µM BRQ.

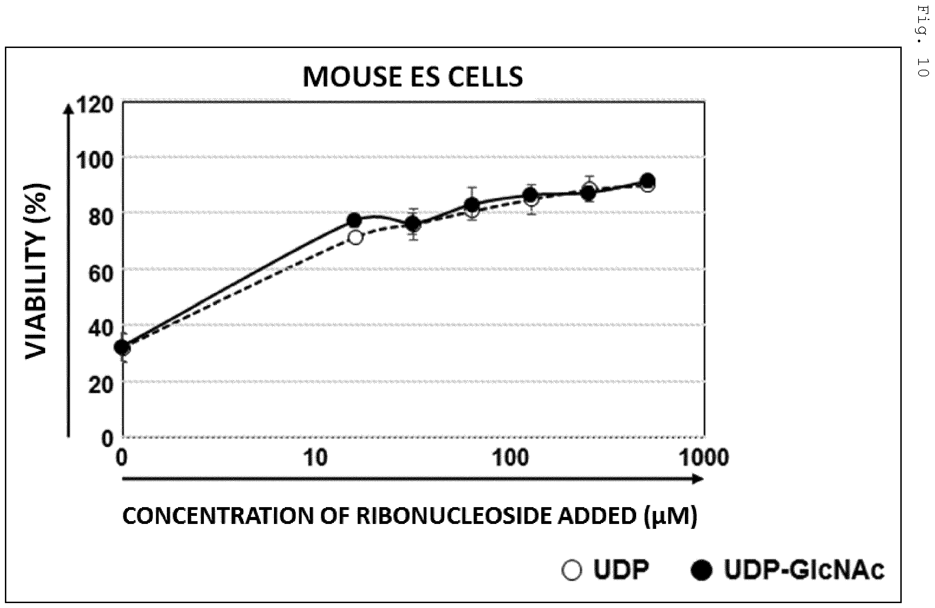

[Fig. 10] Fig. 10 is a graph showing the viability of mouse ES cells when various ribonucleosides (UDP or UDP-GlcNAc) are added in the presence of 10 µM BRQ.

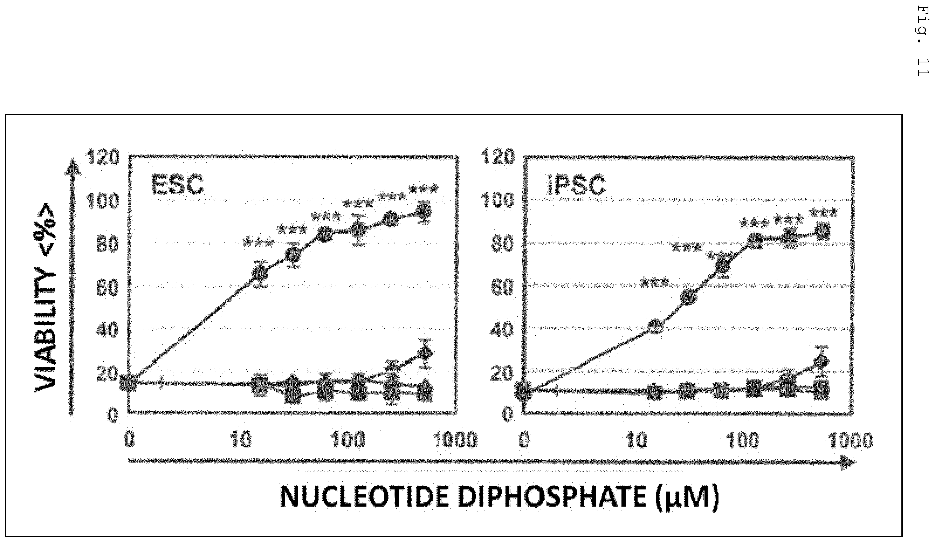

[Fig. 11] Fig. 11 shows the dose-dependent effects of nucleotide diphosphate on BRQ-dependent cytotoxicity in pluripotent stem cells. In this figure, "ESC" indicates the effect on mouse ES cells, and "iPSC" indicates the effect on mouse iPS cells. In each graph, circles indicate the dose-dependent effects of UDP, diamonds indicate those of CDP, squares indicate those of ADP, and triangles indicate those of GDP. Error bars indicate ±SD (standard deviation). Statistical significance was determined by t-test. **p<0.01, ***p<0.001.

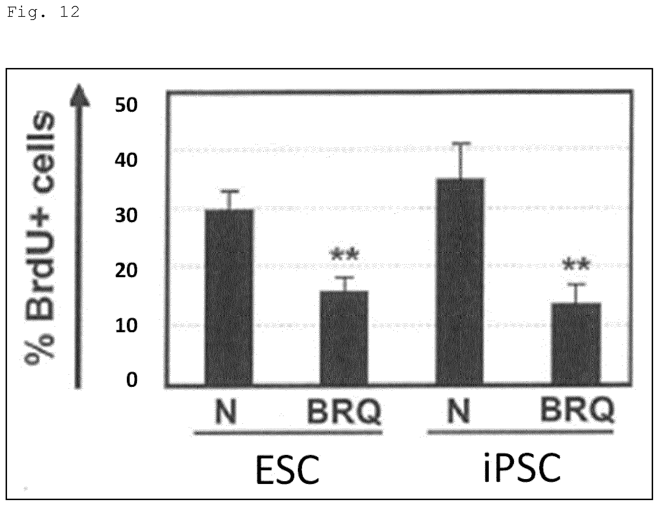

[Fig. 12] Fig. 12 shows the percentage of BrdU-positive cells in pluripotent stem cells in the presence of BRQ. In the figure, "ESC" indicates the above percentage in mouse ES cells, and "iPSC" indicates the above percentage in mouse iPS cells. "N" and "BRQ" indicate the absence and presence of BRQ, respectively, in the culture medium of pluripotent stem cells.

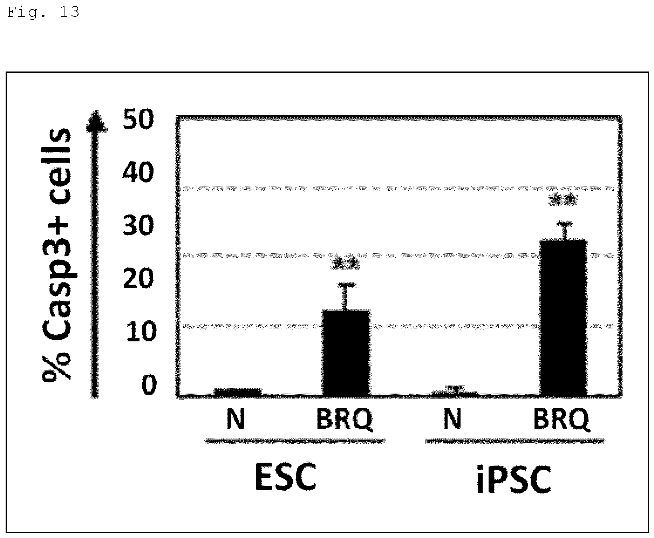

[Fig. 13] Fig. 13 shows the percentage of Casp3-positive cells in pluripotent stem cells in the presence of BRQ. In the figure, "ESC" indicates the above percentage in mouse ES cells, and "iPSC" indicates the above percentage in mouse iPS cells. "N" and "BRQ" indicate the absence and presence of BRQ, respectively, in the culture medium of pluripotent stem cells.

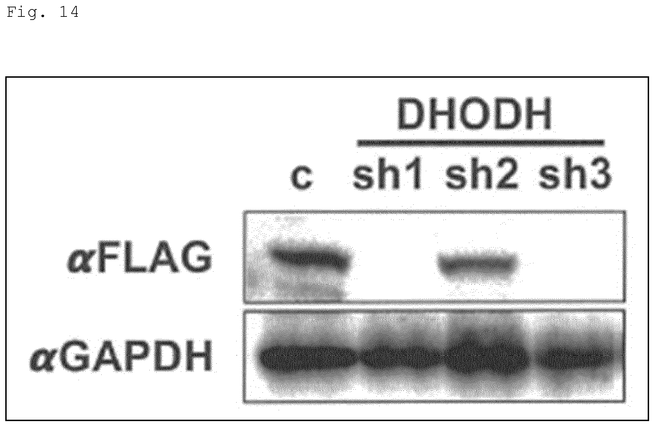

[Fig. 14] Fig. 14 provides photographs showing the results of analyzing knockdown efficiency by DHODH expression vectors by Western blotting. FLAG-tagged mouse DHODH expression vectors were introduced into Cos7 cells along with vectors encoding control shRNA ("C" in the figure) or vectors encoding shRNAs 1 to 3 against DHODH ("DHODH sh1, sh2, sh3" in the figure), respectively. Two days after the gene transfer, cell extracts were collected and analyzed by Western blotting using an anti-FLAG antibody and an anti-GAPDH antibody (loading control).

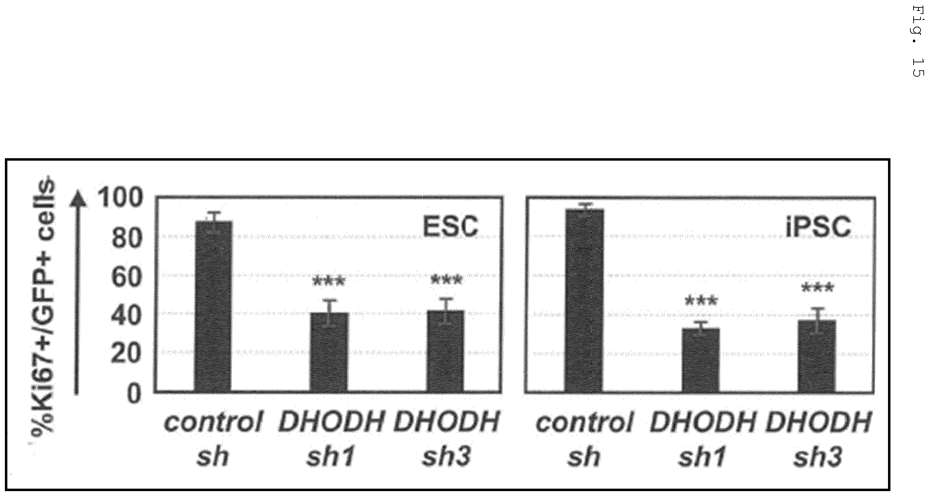

[Fig. 15] Fig. 15 shows the percentage of Ki67-positive cells in control shRNA-expressing pluripotent stem cells or DHODH shRNA-expressing pluripotent stem cells. In the figure, "ESC" and "iPSC" indicate the above percentages for mouse ES cells and mouse iPS cells, respectively. Error bars indicate ±SD. Statistical significance was determined by t-test. *P<0.05, **P<0.01, ***P<0.001.

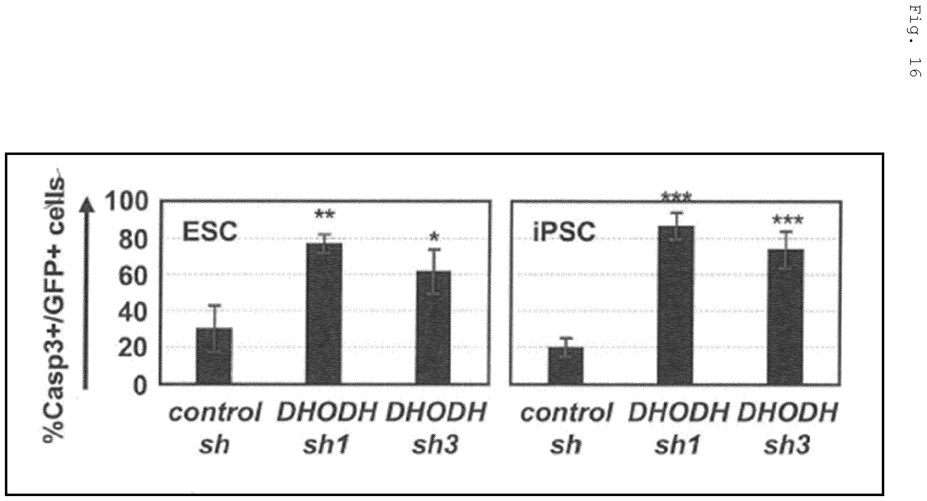

[Fig. 16] Fig. 16 shows the percentage of Casp3-positive cells in control shRNA-expressing pluripotent stem cells or DHODH shRNA-expressing pluripotent stem cells. In the figure, "ESC" and "iPSC" indicate the above percentages for mouse ES cells and mouse iPS cells, respectively. Error bars indicate ±SD. Statistical significance was determined by t-test. *P<0.05, **P<0.01, ***P<0.001.

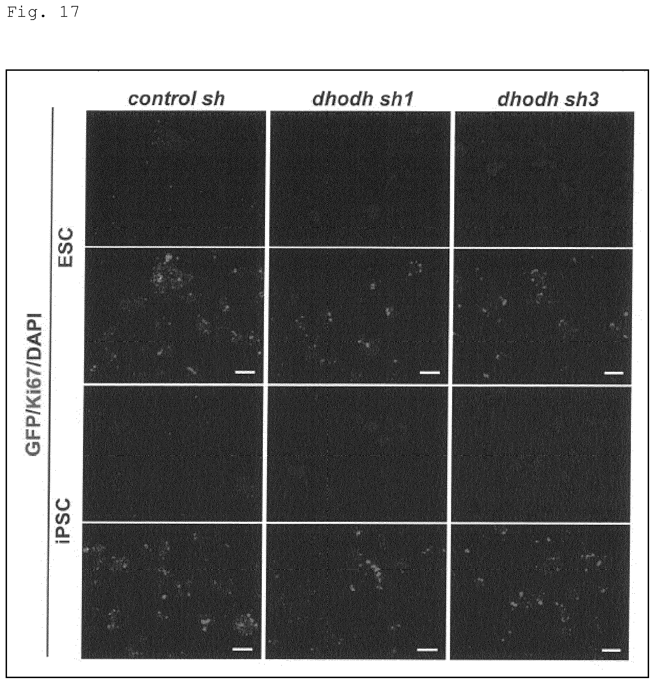

[Fig. 17] Fig. 17 provides fluorescence micrographs showing representative results obtained by immunostaining and observing control shRNA-expressing pluripotent stem cells and dhodh sh-expressing pluripotent stem cells for GFP and Ki67. In the figure, "ESC" and "iPSC" indicate the results of observing mouse ES cells and mouse iPS cells, respectively. The three photographs each in the first and third rows show the results of detecting the expression of Ki67 (red) and nuclei (blue, contrast staining with DAPI) of mouse ES cells and mouse iPS cells. The three photographs each in the second and fourth rows show the results of detecting the expression of GFP (green) and Ki67 (red) of mouse ES cells and mouse iPS cells. The scale bar indicates 100 µm.

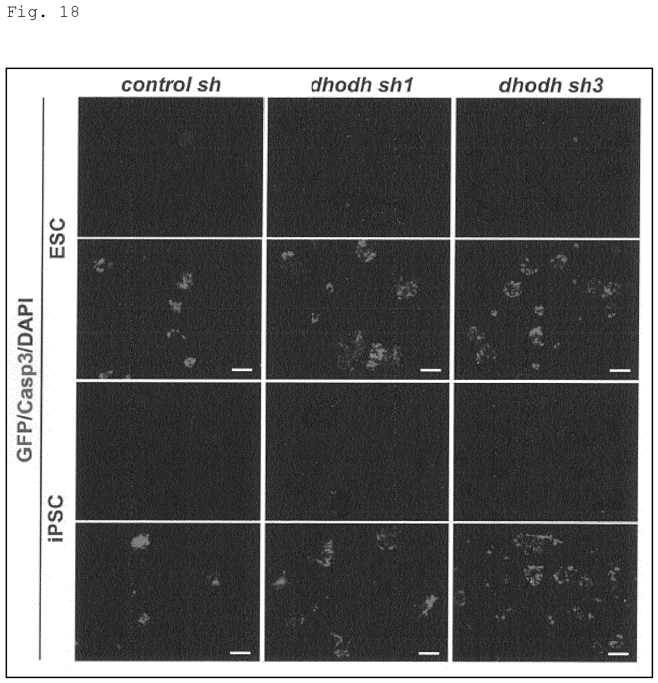

[Fig. 18] Fig. 18 provides fluorescence micrographs showing representative results obtained by immunostaining and observing control shRNA-expressing pluripotent stem cells and dhodh sh-expressing pluripotent stem cells for GFP and Casp3. In the figure, "ESC" and "iPSC" indicate the results of observing mouse ES cells and mouse iPS cells, respectively. The three photographs each in the first and third rows show the results of detecting the expression of Casp3 (red) and nuclei (blue, contrast staining with DAPI) of mouse ES cells and mouse iPS cells. The three photographs each in the second and fourth rows show the results of detecting the expression of GFP (green) and Casp3 (red) of mouse ES cells and mouse iPS cells. The scale bar indicates 100 µm.

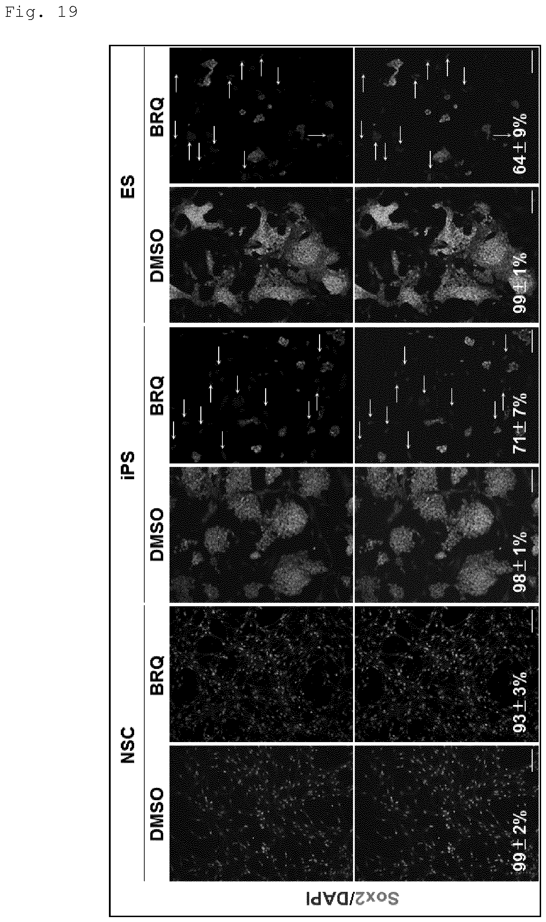

[Fig. 19] Fig. 19 provides photographs showing the results of observing various cells with a fluorescence microscope after 24 hours of culture in the presence of 10 µM BRQ. In the figure, "NSC," "iPS," and "ES" indicate the results of observing mouse neural stem cells, mouse iPS cells, and mouse ES cells. "BRQ" indicates the results of culture in the presence of 10 µM BRQ, and "DMSO" indicates the results of culture in the absence of BRQ. The six photographs in the upper row show the results of Sox2 immunostaining, and the six photographs in the lower row show the results of Sox2 immunostaining, superimposed on contrast staining of cell nuclei with DAPI. The arrows indicate Sox2-negative cells. Each number indicates the percentage (%) of Sox2-positive cells to DAPI-positive cells. The scale bar indicates 100 µm.

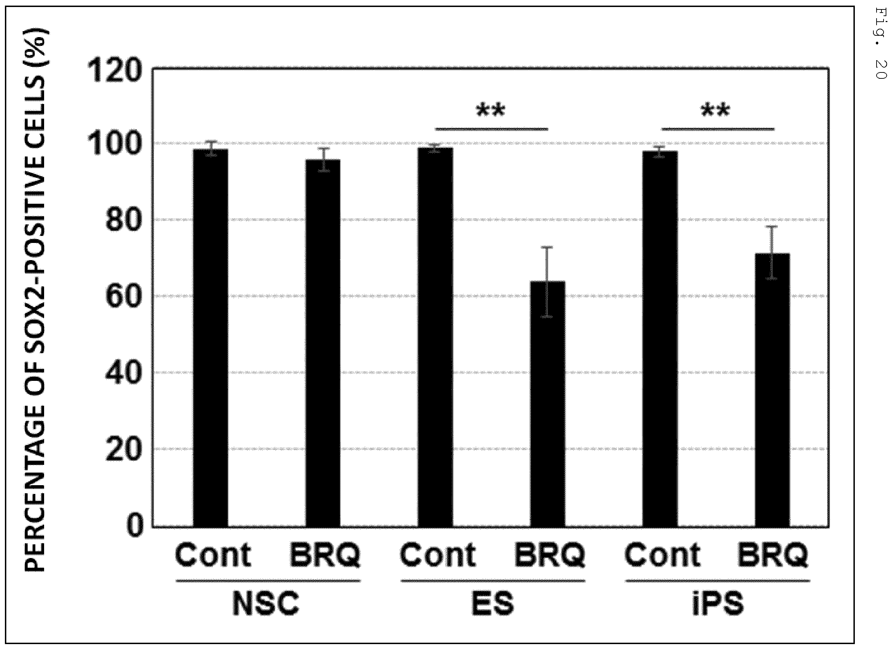

[Fig. 20] Fig. 20 shows the percentage (%) of Sox2-positive cells in various cells after 24 hours of culture in the presence of 10 µM BRQ. In the figure, "NSC," "iPS," and "ES" indicate the percentage of Sox2-positive cells in mouse neural stem cells, mouse iPS cells, and mouse ES cells, respectively. "BRQ" indicates the results of culture in the presence of 10 µM BRQ, and "Cont" indicates the results of culture in the absence of BRQ. The "**" indicates that the P value<0.01.

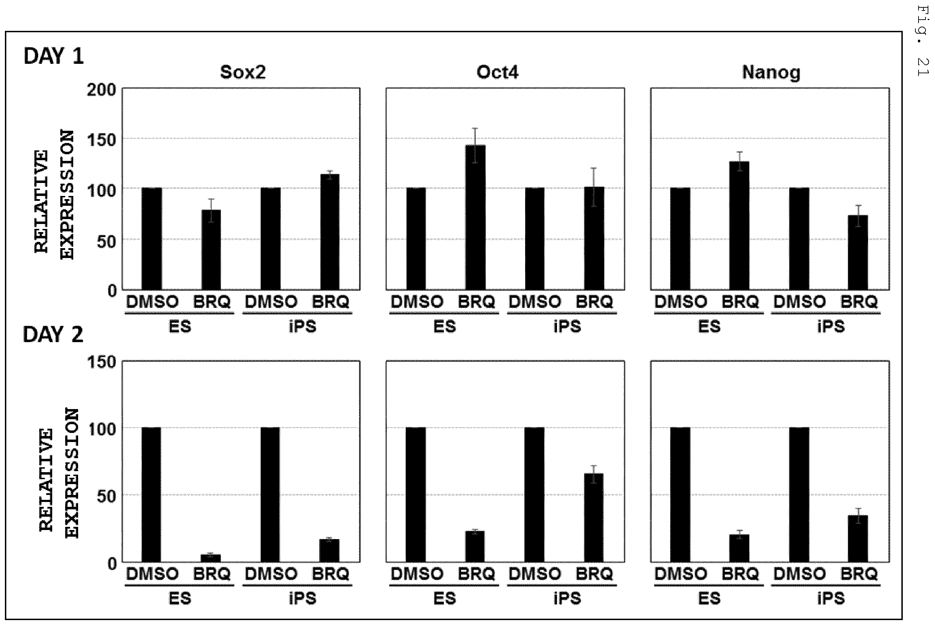

[Fig. 21] Fig. 21 provides graphs showing the expression levels of pluripotent stem cell marker genes (Sox2, Oct4, and Nanog) in various cells after 1 or 2 days of culture in the presence of 10 µM BRQ. In the figure, "ES" and "iPS" indicate the expression level of each gene in mouse ES cells and mouse iPS cells. "BRQ" indicates the results of culture in the presence of 10 µM BRQ, and "DMSO" indicates the results of culture in the absence of BRQ. The vertical axis of each graph shows a relative value when the expression level of each gene in "DMSO" is 100.

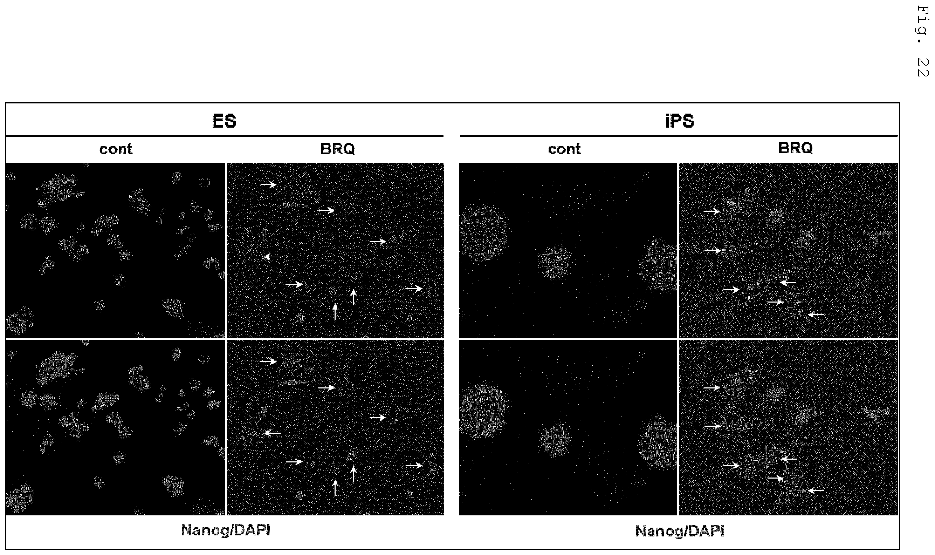

[Fig. 22] Fig. 22 provides photographs showing the results of observing various cells with a fluorescence microscope after 2 days of culture in the presence of 10 µM BRQ. In the figure, "ES" and "iPS" indicate the results of observing mouse ES cells and mouse iPS cells. "BRQ" indicates the results of culture in the presence of 10 µM BRQ, and "cont" indicates the results of culture in the absence of BRQ. The four photographs in the upper row show the results of Nanog immunostaining, and the four photographs in the lower row show the results of Nanog immunostaining, superimposed on the results of contrast staining of cell nuclei with DAPI. The arrows indicate cells in which Nanog is distributed in their cytoplasm (cells in which Nanog has been excluded from their nuclei).

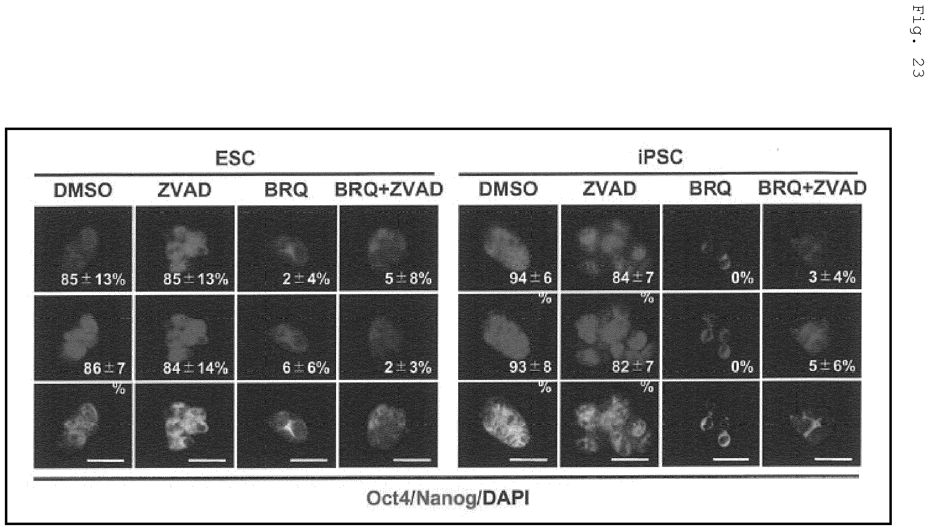

[Fig. 23] Fig. 23 shows fluorescence micrographs of pluripotent stem cells (PSCs) cultured for 2 days in the presence of DMSO, ZVAD (100 nM), BRQ (10 µM), or BRQ and ZVAD (BRQ: 10 µM, ZVAD: 100 nM) and immunolabeled for Nanog and Oct4. In the figure, "ESC" and "iPSC" indicate the results of observing mouse ES cells and mouse iPS cells. The numbers in the figure indicate the percentage of the number of the corresponding nuclear PSC marker-positive cells in the total cells. The eight photographs in the upper row show the results of Oct4 immunostaining (green), the eight photographs in the middle row show the results of Nanog immunostaining (red), and the eight photographs in the lower row show the results of Oct4 and Nanog immunostaining, superimposed on the results of contrast staining of cell nuclei with DAPI (blue). The scale bar indicates 50 µm.

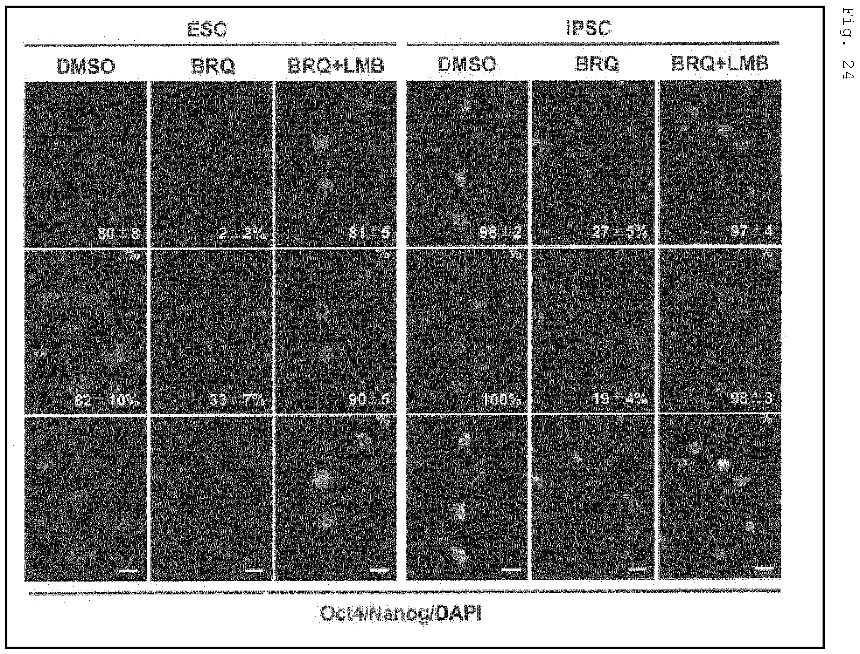

[Fig. 24] Fig. 24 shows fluorescence micrographs of pluripotent stem cells (PSCs) cultured for 2 days in the presence of DMSO, BRQ (10 µM), or BRQ and LMB (BRQ: 10 µM, LMB: 0.15 nM) and immunolabeled for Nanog (red) and Oct4 (green). In the figure, "ESC" and "iPSC" indicate the results of observing mouse ES cells and mouse iPS cells, respectively. The numbers in the figure indicate the percentage of the number of the corresponding nuclear PSC marker-positive cells in the total cells. The six photographs in the upper row show the results of Oct4 immunostaining (green), the six photographs in the middle row show the results of Nanog immunostaining (red), and the six photographs in the lower row show the results of Oct4 and Nanog immunostaining, superimposed on the results of contrast staining of cell nuclei with DAPI (blue). The scale bar indicates 100 µm.

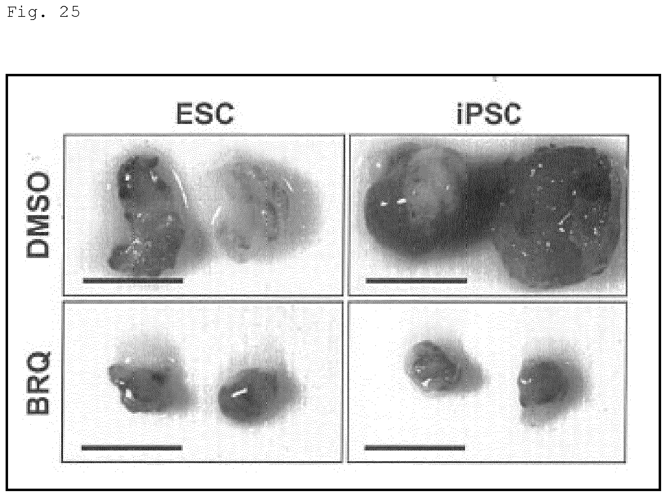

[Fig. 25] Fig. 25 shows representative photographs of teratomas formed from pluripotent stem cells pretreated with DMSO or BRQ. In the figure, "ESC" and "iPSC" indicate the results of observing teratomas formed from mouse ES cells and mouse iPS cells, respectively. The scale bar indicates 1 cm.

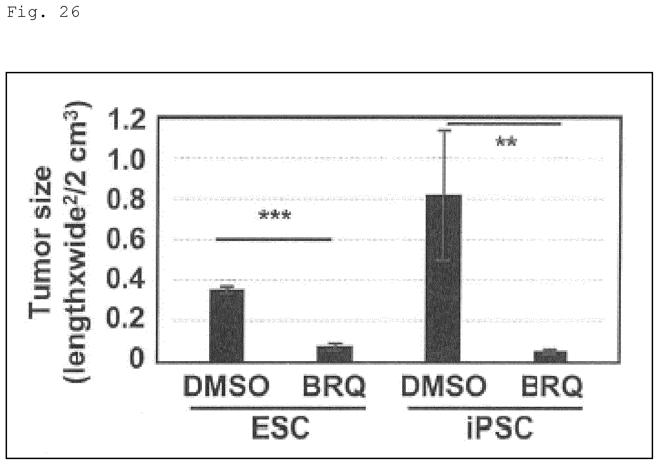

[Fig. 26] Fig. 26 shows the volume of tumors formed from pluripotent stem cells pretreated with DMSO or BRQ. In the figure, "ESC" and "iPSC" indicate the volume of tumors formed from mouse ES cells and mouse iPS cells, respectively. Error bars indicate ±SD. Statistical significance was determined by t-test. **P<0.01, ***P<0.001.

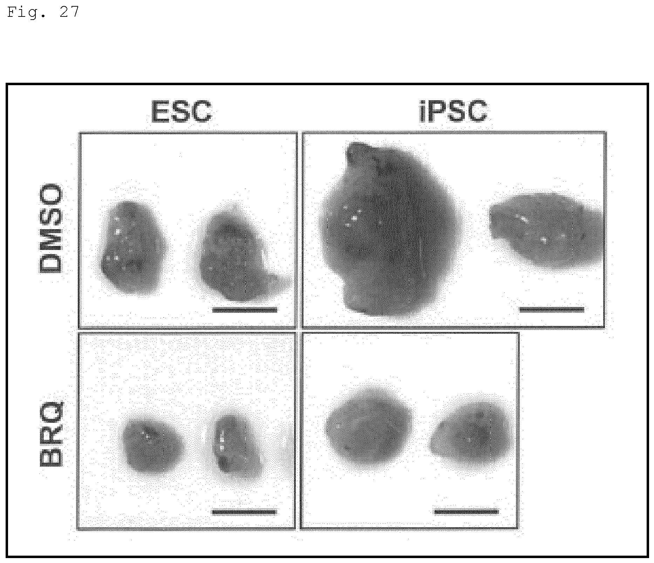

[Fig. 27] Fig. 27 shows representative photographs of teratomas formed from pluripotent stem cells in mice treated with DMSO or BRQ. In the figure, "ESC" and "iPSC" indicate the results of observing teratomas formed from mouse ES cells and mouse iPS cells, respectively. The scale bar indicates 1 cm.

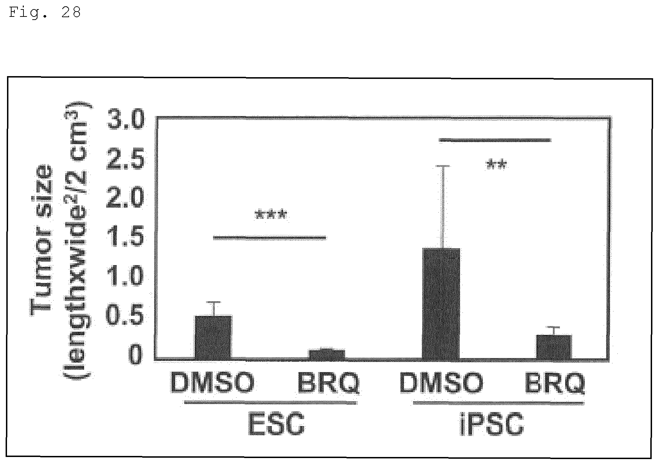

[Fig. 28] Fig. 28 shows the tumor volume of teratomas formed from pluripotent stem cells in mice treated with DMSO or BRQ. In the figure, "ESC" and "iPSC" indicate the volume of tumors formed from mouse ES cells and mouse iPS cells, respectively. Error bars indicate ±SD. Statistical significance was determined by t-test. **P<0.01, ***P<0.001.

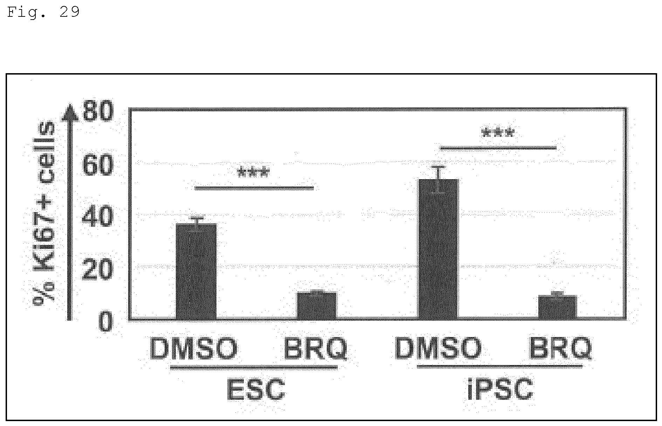

[Fig. 29] Fig. 29 shows the percentage of Ki67-positive cells in teratomas formed from pluripotent stem cells in mice treated with DMSO or BRQ. In the figure, "ESC" and "iPSC" indicate the above percentages in teratomas formed from mouse ES cells and mouse iPS cells, respectively. Error bars indicate ±SD. Statistical significance was determined by t-test. **P<0.01, ***P<0.001.

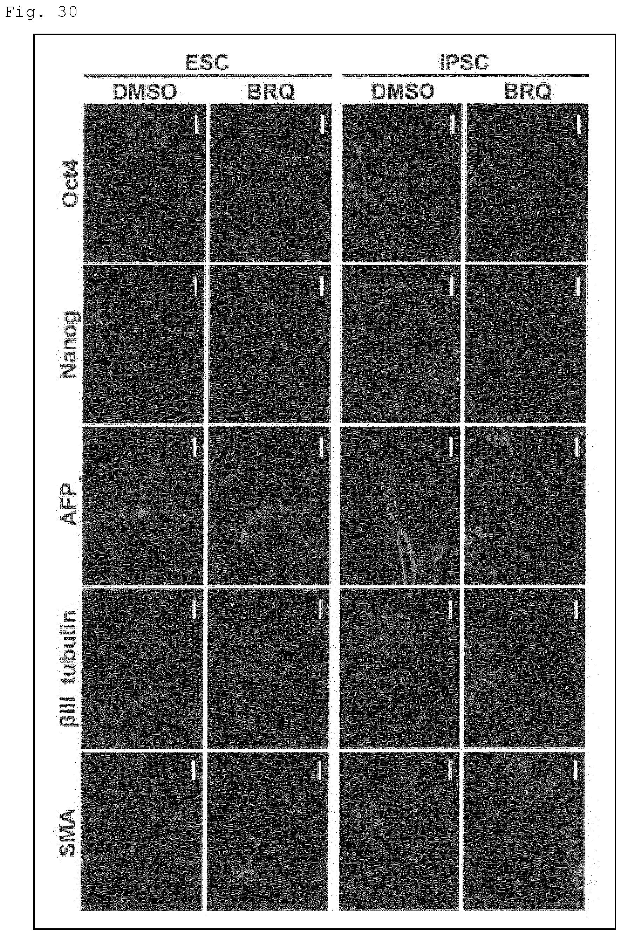

[Fig. 30] Fig. 30 shows representative images of teratomas immunostained for Oct4 (red), Nanog (green), AFP (green), βIII tubulin (red), and SMA (green). In the figure, "ESC" and "iPSC" represent teratomas formed from mouse ES cells and mouse iPS cells, respectively. Nuclei were detected by contrast staining with DAPI (blue). The scale bar indicates 100 µm.

### [Description of Embodiments]

As shown in Examples described later, the present inventor has shown that inhibiting the enzymatic activity of dihydroorotate dehydrogenase (DHODH) and inhibiting pyrimidine synthesis can cause cytotoxicity to pluripotent stem cells and eliminate the cells. On the other hand, it has been found that inhibition of DHODH does cause significant cytotoxicity in differentiated cells such as somatic stem cells and somatic cells obtained by inducing differentiation of pluripotent stem cells.

Therefore, the present invention provides a composition and a method for eliminating undifferentiated pluripotent stem cells remaining in a cell group induced to differentiate from pluripotent stem cells using a DHODH inhibitor.

In the present invention, "dihydroorotate dehydrogenase (DHODH)" means an enzyme that catalyzes the oxidation of dihydroorotate to orotate, which is the fourth reaction in the de novo synthetic pathway of pyrimidine, and examples thereof include dihydroorotate dehydrogenase (fumarate) (EC number: 1.3.98.1), dihydroorotate dehydrogenase (NAD+) (EC number: 1.3.1.14), dihydroorotate dehydrogenase (NADP+) (EC number: 1.3.1.15), and dihydroorotate dehydrogenase (quinone) (EC number: 1.3.5.2).

The "dihydroorotate dehydrogenase inhibitor <DHODH inhibitor)" means a compound having an activity of inhibiting the above catalytic reaction. In addition, "inhibition" in the present invention includes not only complete inhibition of activity and the like but also partial inhibition (suppression).

Examples of the "DHODH inhibitor" of the present invention include brequinar (BRQ, 6-fluoro-2-(2'-fluoro-1,1'-biphenyl-4-yl)-3-methyl-4-quinoline-sodium carboxylate), leflunomide (5-methyl-N-[4-(trifluoromethyl)phenyl]-isoxazole-4-carboxamide), teriflunomide ((2Z)-2-cyano-3-hydroxy-N-[4-(trifluoromethyl)phenyl]but-2-enamide), and vidofludimus (2-(3-fluoro-3'-methoxybiphenyl-4-ylcarbamoyl)-cyclopenta-1-ene carboxylic acid, ASLAN003 (2-(3,5-difluoro-3'methoxybiphenyl-4-ylamino)nicotinic acid), as shown in Examples to be described later. Further, examples include compounds 15 to 110 as disclosed in J Med Chem., 2013 Apr 25; 56(8): 3148-67, compounds 7a to 7n as disclosed in Eur J Med Chem. 2012 Mar; 49: 102-9, compound codes 3 to 8 as disclosed in Biochemistry. 2008 Aug 26; 47 (34): 8929-36, aminonicotinic and isonicotinic acid derivatives as disclosed in International Publication No. 2008/077639, and compound 10580 (IC₅₀: 9 nM as described later) disclosed in Neuro Oncol. 2019 Sep 10. pii: noz170. doi: 10.1093/neuonc/noz170.

As the "DHODH inhibitor" of the present invention, a compound with a 50% inhibitory concentration (IC₅₀) against human-derived DHODH of 100 nM or less is preferable, a compound with an IC₅₀ of 50 nM or less is more preferable, and a compound with an IC₅₀ of 20 nM or less is further preferable. Examples of such compounds include the following compounds (the structure of each compound and the IC₅₀ for human-derived DHODH are shown below).

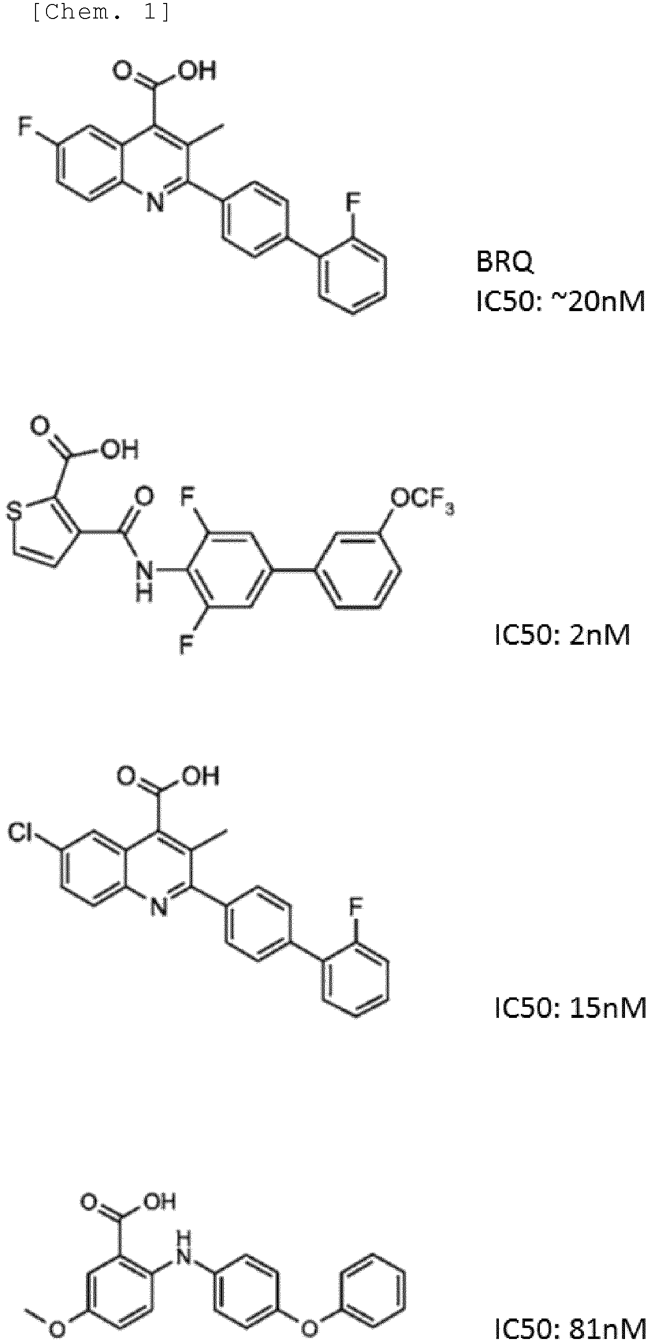

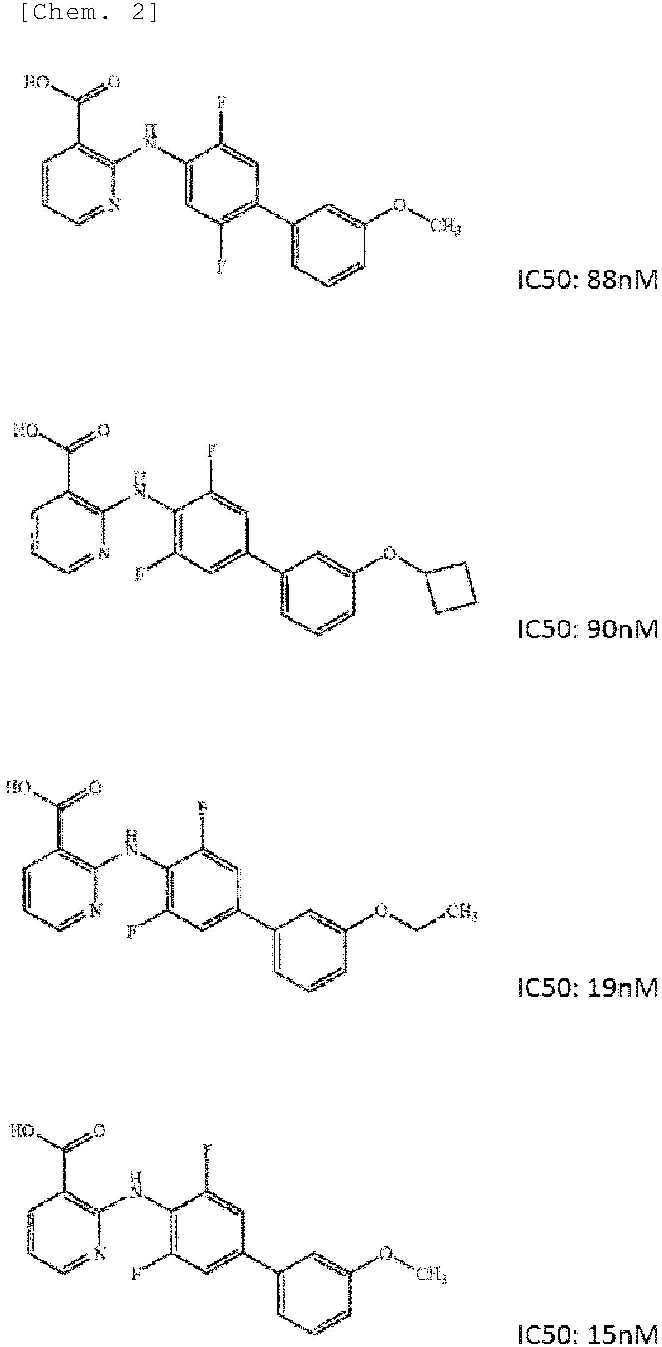

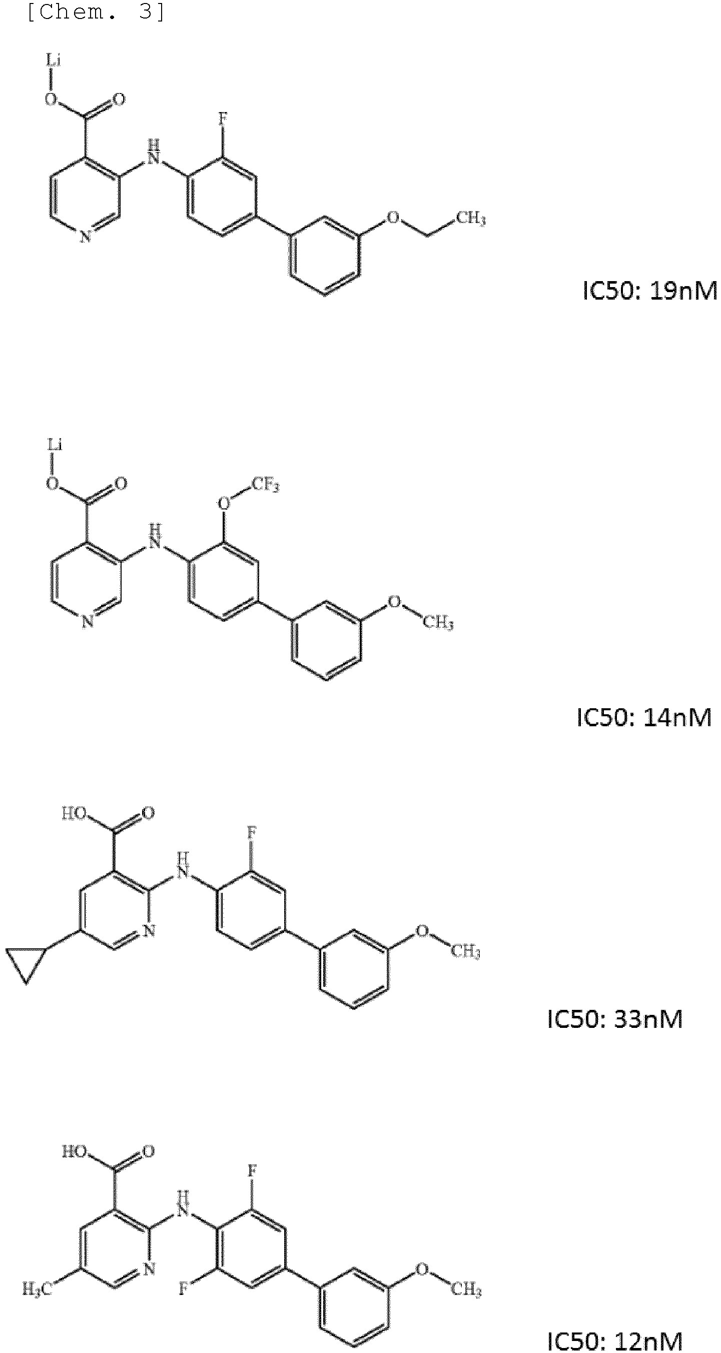

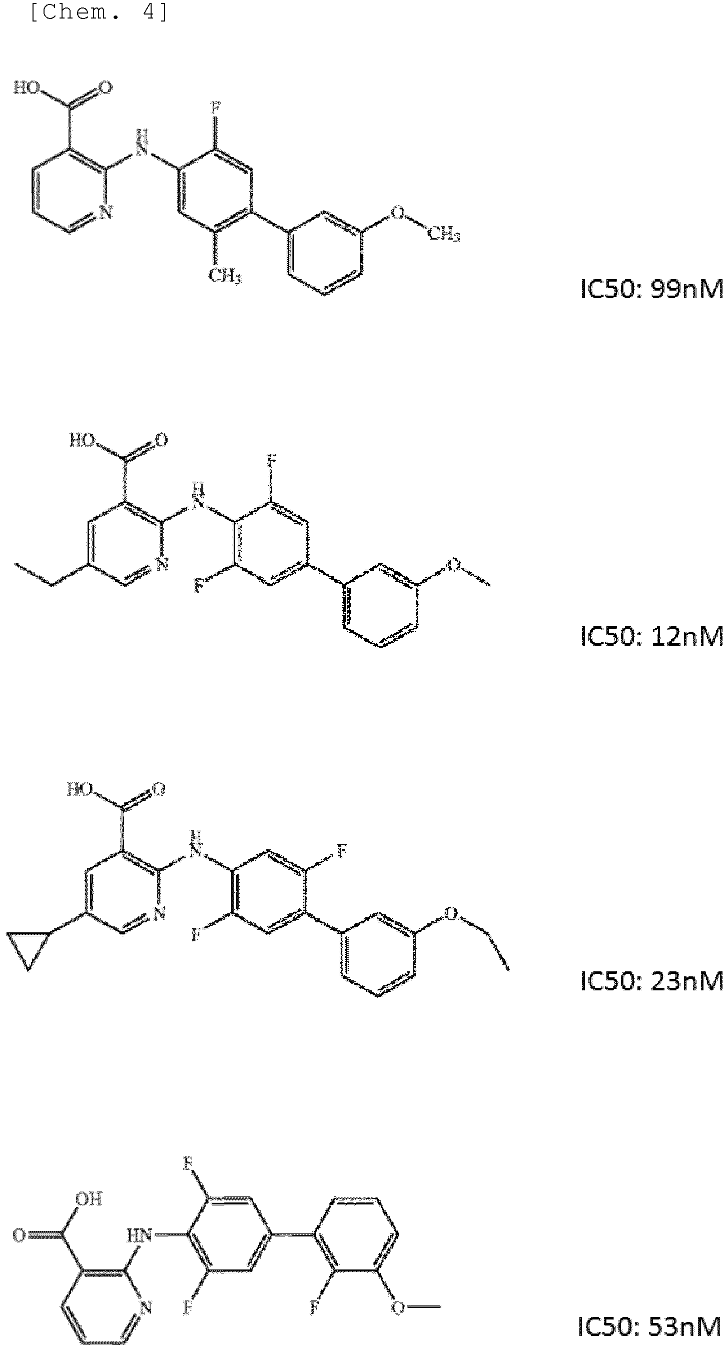

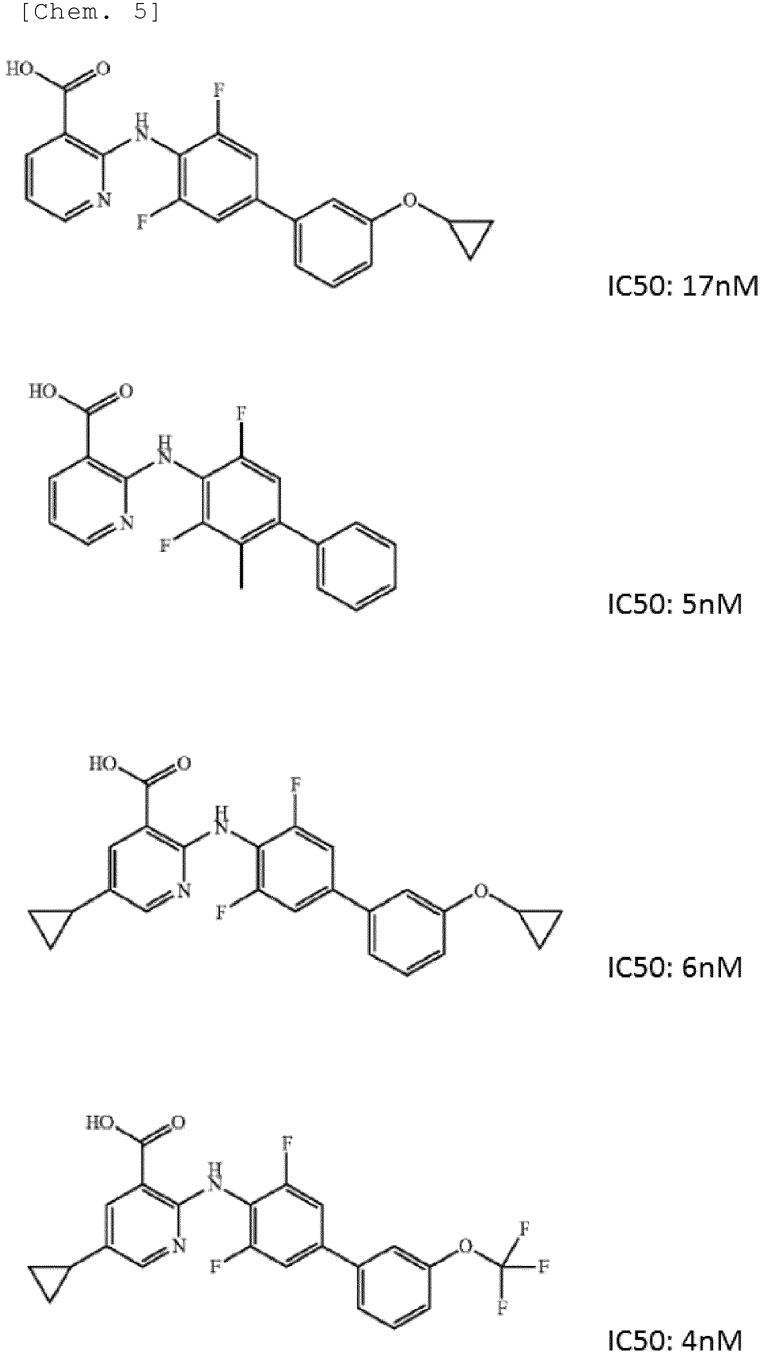

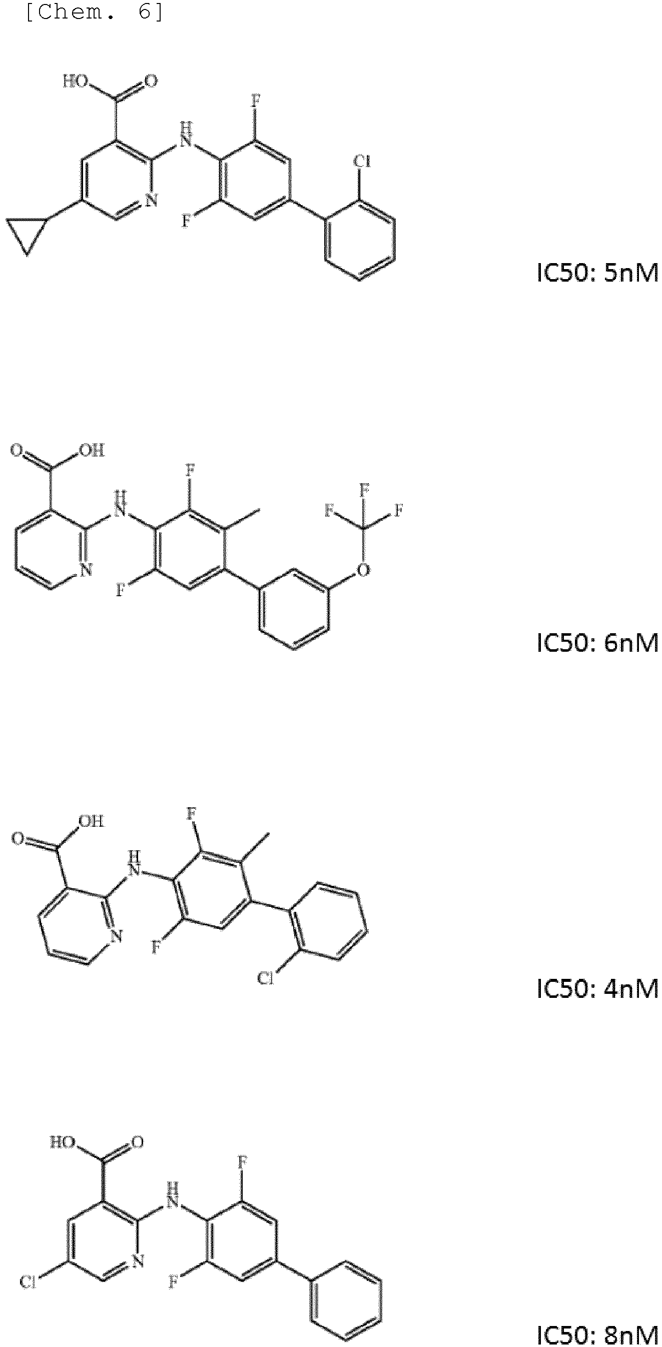

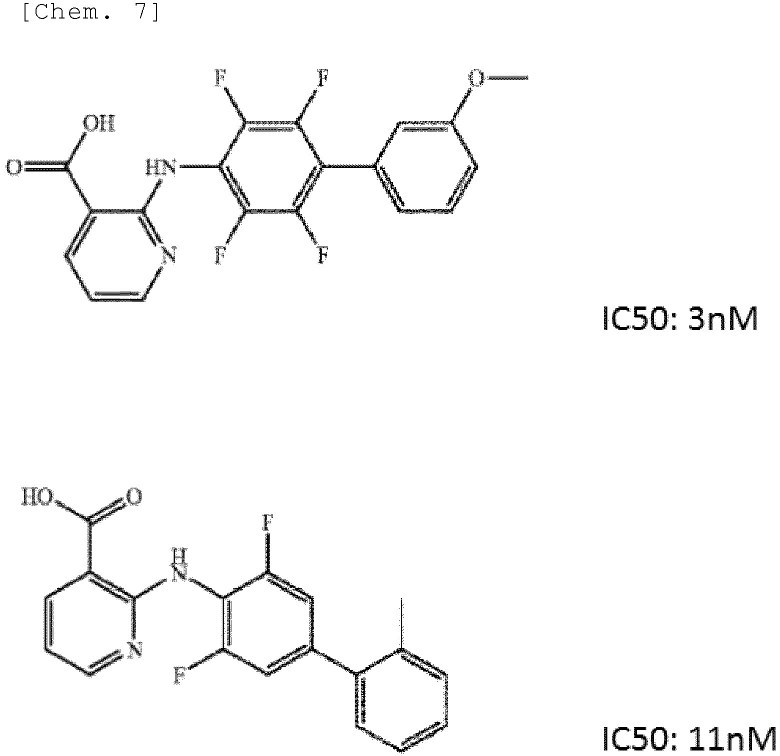

The enzymatic activity of DHODH can be determined by those skilled in the art, for example, by a dye reduction assay using dichloroindophenol (DCIP). DCIP having an absorption maximum of 600 nm is reduced and becomes colorless as the substrate is oxidized by DHODH and the electron acceptor is reduced. Therefore, the activity of DHODH can be determined by using the decrease in absorbance at the wavelength as an index. In addition, the IC₅₀ for DHODH can be determined by adding the test compound (DHODH inhibitor) at various concentrations to the enzyme reaction solution containing, for example, human-derived DHODH, substrate (dihydroorotate), electron acceptor (such as CoQ), and buffer solution, and performing the DCIP assay described above (see International Publication No. 2008/077639, Biochem J. 1998 Dec 1; 336 (Pt2): 299-303).

The "DHODH inhibitor" according to the present invention also includes a pharmacologically acceptable salt, hydrate, or solvate as long as it has an inhibitory activity thereof. The pharmacologically acceptable salt is not particularly limited and may be appropriately selected depending on the structure of the drug and the like, and examples thereof include acid addition salts (such as hydrochlorides, sulfates, hydrobromides, nitrates, hydrogen sulfates, phosphates, and acetates) and base addition salts (such as sodium salts, potassium salts, zinc salts, and calcium salts). In addition, the hydrate or solvate is not particularly limited, and examples thereof include those obtained by adding 0.1 to 10 molecules of water or a solvent to one molecule of a DHODH inhibitor or a salt thereof.

Furthermore, the "DHODH inhibitor" according to the present invention includes all isomers and isomer mixtures, such as tautomers, geometric isomers, optical isomers based on asymmetric carbons, and stereoisomers, as long as they have the inhibitory activity. The present invention also includes compounds in which the DHODH inhibitor undergoes metabolism in vivo, such as oxidation, reduction, hydrolysis, amination, deamination, hydroxylation, phosphorylation, dehydroxylation, alkylation, dealkylation, and conjugation, and still exhibits its inhibitory activity, and also includes compounds that undergo metabolism in vivo, such as oxidation, reduction, and hydrolysis, to produce a DHODH inhibitor.

Note that as for DHODH inhibitors, as mentioned above, many compounds have been developed and are commercially available, and thus can be obtained by purchasing them. Even if they are not commercially available, there are many reports on how to produce these compounds. Therefore, those skilled in the art can prepare them appropriately according to the production methods.

In the present invention, "pluripotent stem cells" can be any cells that have differentiation pluripotency and self-renewal capability, and examples thereof include cells that can be harvested from living organisms, such as embryonic stem cells (ES cells), embryonal carcinoma cells (EC cells), epiblast stem cells (EpiS cells), embryonic germ cells (EG cells), multipotent germline stem cells (mGS cells), and MUSE cells (see Kuroda Y. et al., Proc. Natl. Acad. Sci. U.S.A., 2010, Vol. 107, Issue 19, pp. 8639-8643). Furthermore, the "pluripotent stem cells" also include cells that have been artificially induced to have pluripotency from somatic cells harvested from living organisms, such as induced pluripotent stem cells (iPS cells). In addition, there are no particular restrictions on the origin of these cells, and examples thereof include human and non-human animals (for example, rodents such as mice and rats, mammals such as cattle, horses, pigs, sheep, monkeys, dogs, and cats, and birds such as chickens). Furthermore, for the purpose of regenerative medicine and the like to be described later, it is desirable to use pluripotent stem cells derived from the target of transplantation of the differentiated cells described later from the viewpoint of suppressing immune rejection.

In the present invention, "undifferentiated pluripotent stem cells" include not only the aforementioned pluripotent stem cells that have not differentiated and maintain differentiation pluripotency, but also cells in which pluripotent stem cells have become tumors (such as teratomas and teratocarcinomas).

In the present invention, a "cell group induced to differentiate from pluripotent stem cells" means a population of cells that includes any differentiated cells derived from the pluripotent stem cells described above. Such a cell group may include a single species of differentiated cells or multiple species of differentiated cells. Furthermore, it may be a tissue or an organ composed of such cells as a form thereof.

In the present invention, "differentiated cells" include not only cells that have completely lost their pluripotency (final differentiated cells such as somatic cells and germ cells), but also cells that have partially lost their pluripotency (such as progenitor cells and somatic stem cells) . The "somatic stem cells" are cells also referred to as adult stem cells or tissue stem cells, and examples thereof include neural stem cells, satellite cells, hematopoietic stem cells (bone marrow stem cells), mesenchymal stem cells, intestinal stem cells, hair follicle stem cells, mammary stem cells, endothelial stem cells, olfactory mucosa stem cells, neural cortex stem cells, and testicular cells.

Induction of differentiation from pluripotent stem cells into "differentiated cells" is usually done through induction of differentiation into the germ layers (ectoderm, mesoderm, and endoderm) in which each differentiated cell develops, and those skilled in the art can appropriately select a known method using low-molecular-weight compounds, proteins, and the like (differentiation-inducing factors) that are suitable for inducing differentiation into each germ layer-type cell. For example, ectodermal cells such as neurons can be induced to differentiate by culturing them in the presence of BMP inhibitors, TGFβ, and activin inhibitors (see Nat Biotechnol. 2009 Mar; 27(3): 275-80). For example, pluripotent stem cells can be induced to differentiate into mesodermal cells such as chondrocytes by culturing them in the presence of Wnt and activin, and then culturing them in the presence of BMP4 (see Development. 2008 Sep; 135(17): 2969-79) . Moreover, for example, endoderm cells such as pancreatic cells and intestinal cells can be induced to differentiate by culturing pluripotent stem cells in the presence of activin and then culturing them in the presence of BMP4 (for example, see Diabetes 2010 Sep; 59(9): 2094-2101, Nature. 2011 Feb 3; 470 (7332) : 105-9) .

In the present invention, the "elimination" of undifferentiated pluripotent stem cells remaining in the cell group described above means not only the complete elimination of such cells, but also a reduction in the number or percentage of such cells in the cell group. Such elimination may be performed in vitro, in vivo, or ex vivo.

### (Composition for Eliminating Pluripotent Stem Cells)

An embodiment of the present invention is a composition for eliminating undifferentiated pluripotent stem cells remaining in a cell group induced to differentiate from pluripotent stem cells, comprising: a DHODH inhibitor as an active ingredient.

In addition to the DHODH inhibitor described above, the composition of the present invention may also contain a physiologically acceptable carrier. Examples of the physiologically acceptable carrier include physiological isotonic solutions (such as isotonic solutions containing saline, culture media, dextrose, and other adjuvants (such as D-sorbitol, D-mannitol, and sodium chloride)), excipients, antiseptics, stabilizers (such as human serum albumin and polyethylene glycol), binding agents, dissolution aids, nonionic surfactants, buffers (such as phosphate buffer solution, and sodium acetate buffer solution), preservatives, and antioxidants.

The composition of the invention can be in the form of a reagent used for research or medical purposes (for example, production of differentiated cells, especially differentiated cells with reduced risk of tumorigenesis). It can also be in the form of a pharmaceutical composition that is administered to a subject transplanted with the differentiated cells, as described below.

Also, the present invention also provides a kit for producing differentiated cells, especially differentiated cells with reduced risk of tumorigenesis. In addition to the aforementioned composition, the kit can include materials necessary for inducing differentiation of pluripotent stem cells into desired cells, such as pluripotent stem cells, differentiation-inducing factors as described above, differentiation-inducing medium, and culture vessels. In addition, reagents for detecting such cell-specific marker molecules (such as antibodies specific for such marker molecules) to confirm that differentiation into desired cells has been induced can also be included in the kits of the present invention. Also included in the kit of the present invention is an instruction manual showing how to use the composition of the present invention, how to induce differentiation, and the like.

### (Method for Eliminating Pluripotent Stem Cells)

Another embodiment of the present invention is a method for eliminating undifferentiated pluripotent stem cells remaining in a cell group, comprising: contacting a cell group induced to differentiate from pluripotent stem cells with a dihydroorotate dehydrogenase inhibitor.

There are no particular restrictions on the "contact" between the DHODH inhibitor and the cell group described above, and this can be done, for example, by adding the DHODH inhibitor to the medium for maintaining the cell group. The concentration of addition is not particularly limited and can be adjusted by those skilled in the art depending on the type of pluripotent stem cells and cell group to be treated and the type of DHODH inhibitor to be used, and is usually 0.1 to 1000 µM, preferably 1 to 100 µM, and further preferably 10 to 50 µM.

By eliminating undifferentiated pluripotent stem cells in this manner, it is possible to obtain differentiated cells with reduced risk of tumorigenesis, which are useful for regenerative medicine and the like. Therefore, the method of the present invention can also be rephrased as a "method for producing differentiated cells with reduced risk of tumorigenesis, comprising: contacting a cell group induced to differentiate from pluripotent stem cells with a dihydroorotate dehydrogenase inhibitor."

When the above differentiated cells are transplanted into a subject (human or non-human animal) for regenerative medicine or the like, the DHODH inhibitor can be administered to the subject to make "contact" between the DHODH inhibitor and the cell group as described above. There are no restrictions on the form of administration, including intravenous administration, intra-arterial administration, intraperitoneal administration, subcutaneous administration, intradermal administration, intratracheal administration, rectal administration, intramuscular administration, infusion administration, topical administration, and oral administration, which are appropriately selected according to the type of cell group to be transplanted, the transplantation site, and the like. In addition, the dosage may be appropriately adjusted according to the type, age, weight, symptoms, and health condition of the subject, form of administration, type of pluripotent stem cells and cell group to be treated, type of DHODH inhibitor to be used, and the like, and the amount per dose is usually 0.001 to 1000 mg/kg body weight, preferably 0.1 to 100 mg/kg body weight, and more preferably 1 to 50 mg/kg body weight. The number of times the drug is administered per day is also not limited, and can be appropriately adjusted, taking into consideration the various factors mentioned above. Furthermore, administration to the subject may be done continuously (for example, once every week).

The contact time between the DHODH inhibitor and the cell group to eliminate undifferentiated pluripotent stem cells can be appropriately adjusted by those skilled in the art as long as the time is sufficient for the elimination, and it is usually 2 to 7 days, preferably 2 to 5 days, and more preferably 2 to 3 days.

### [Examples]

Hereinafter, the present invention is described in more detail based on Examples, but the present invention is not limited to the following Examples. In addition, Examples were carried out using the following materials and methods.

### (Animals and Chemical Reagents)

All experiments using mice were performed in accordance with the protocol approved by the Animal Experiment Committee of Hokkaido University. Mice were purchased from Hokudo Co., Ltd. Unless otherwise noted, chemicals and growth factors were purchased from Invitrogen and PeproTech, respectively.

### (Pluripotent Stem Cells)

The mouse ES cells used were a cell line (E14tg2a) acquired from Shinji Masui (who was working at the Center for iPS Cell Research and Application, Kyoto University, at the time of acquisition of the cells). The mouse iPS cells used were a cell line (iPS-Hep-FB/Ng/gfp-103c-1) purchased from the RIKEN BioResource Research Center.

These pluripotent stem cells were cultured using the method published on SIGMA's website (see https://www.sigmaaldrich.com/life-science/stem-cell-biology/stem-cell-protocols.html). For the culture, a culture medium with the following composition (ESC culture medium) was used.

Glasgow Minimum Essential Medium (GMEM medium, manufactured by Sigma, product number: G5154-500ML) containing 1 mM sodium pyruvate (manufactured by Gibco, catalog number: 11360-070), 1 x non-essential amino acid (manufactured by Gibco, catalog number: 11140-050), 100 µM 2-mercaptoethanol (manufactured by SIGMA, CAS number: 60-24-2), 1000 units/mL leukemia inhibitory factor (LIF, ESGRO (registered trademark), manufactured Gibco, catalog number: ESG1107), 15% KnockOut Serum Replacement (KSR, manufactured by Thermo Fisher Scientific, catalog number: 10828028), and 1% fetal bovine serum (FBS, manufactured by Hyclone). In addition, to maintain these cells in culture, a 100 mm culture dish (manufactured by Falcon) was used, which had been coated on its surface with bovine epidermis-derived gelatin (manufactured by Sigma, product number: G9391) and then placed at 37°C for at least 2 hours.

Human embryonal carcinoma (EC) cells) (NT2) were acquired from the American Tissue Culture Collection (CRL-1973) and cultured in DMEM (Dulbecco's modified Eagle's medium) containing 10% FBS.

### (Differentiated Cells)

Mouse neural stem cells (mNSC) were prepared from mouse (fetal day 14.5) telencephalon as described in Johe KK et al., Genes Dev. 1996, Vol. 10, Issue 24, pp. 3129 to 3140, and were cultured in NSC culture solution [DMEM/F12 medium (manufactured by Sigma) containing bFGF (10 ng/ml), EGF (10 ng/ml), heparin (5 µM), GlutaMAX (manufactured by Gibco), penicillin, and streptomycin].

Mouse bone marrow-derived stromal cells (PA6) were acquired from the RIKEN BioResource Research Center (RBRC-RCB1127) and cultured in αMEM (Minimum Essential Medium Eagle, α Modification) containing 10% FBS.

Mouse myoblasts (C2C12) were acquired from the RIKEN BioResource Research Center (RBRC-RCB0987) and cultured in DMEM containing 10% FBS.

Astrocytes were prepared by culturing mNSCs for 3 days in the presence of DMEM containing 10% FBS, and then maintained in the same medium.

### (MTT Assay Method)

One thousand cells were seeded into each well of a 96-well plate and cultured for 3 days in medium (200 µL/well) supplemented with only DMSO at a final concentration of 0.1% or in medium (200 µL/well) supplemented with each dihydroorotate dehydrogenase (DHODH) inhibitor. The medium used was the appropriate culture solution for each cell as described above.

As a DHODH inhibitor, brequinar (BRQ, Cayman Chemical, CAS number: 96187-53-0), leflunomide (Cayman Chemical, CAS number: 75706-12-6), teriflunomide (Cayman Chemical, CAS number: 163451-81-8), or vidofludimus (Cayman, CAS number: 717824-30-1) was added to the medium. The concentration of each DHODH inhibitor added to the medium was a 2-fold dilution series starting from 200 µM.

As a background, a well containing medium only was prepared, and as a control, a well was also prepared in which medium containing Triton X-100 (final concentration 0.2%) was added to the cells.

Then, 5 µL of MTT (5 mg/ml, manufactured by Wako) was added to each well, and the cells were incubated at 37°C for 2 to 3 hours. Then, the medium was replaced with 100 µL of DMSO to lyse the cells, and the absorbance of these cell lysates at a wavelength of 570 nm was measured with a microplate reader (manufactured by Bio-Rad, Benchmark model). In addition, based on the obtained absorbance, the cell viability was calculated by the following formula.

Cell viability (%)=(absorbance in wells with DHODH inhibitor-absorbance in background)/(absorbance in control-absorbance in background)×100.

### (Nucleoside Neutralization Experiment)

ES cells or iPS cells were incubated for 3 days in the presence of BRQ (10 µM) or in the presence of BRQ (10 µM) and ribonucleosides at various concentrations (up to 500 µM, all manufactured by Sigma), and cell viability was analyzed by the MTT assay described above.

### (Immunostaining)

The cells were fixed and immunostained as described in Kondo et al., Genes Dev. 2004, Vol. 18, Issue 23, pp. 2963-72. Specifically, for immunostaining, the cells were first seeded on coated 8-well chamber slides (glass) and cultured in the presence of 10 µM BRQ for 24 hours. For the culture of mNSCs, PDL+fibronectin-coated ones were used (see Johe KK et al., Genes Dev. 1996, Vol. 10, Issue 24, pp. 3129 to 3140). For the culture of ES cells and iPS cells, gelatin-coated ones were used.

For immunostaining, the cells were first washed once with PBS, then fixed by adding 4% paraformaldehyde and incubating for 10 minutes at room temperature. The cells were replaced with PBS containing 0.3% Triton X100 and incubated for 5 minutes at room temperature, then replaced with PBS containing 0.3% Triton X100 and 10% FCS (blocking solution) and incubated for 30 minutes at room temperature. The solution was replaced with primary antibody solution diluted in blocking solution and incubated for 2 hours at room temperature. After washing three times with PBS, secondary antibody and DAPI solution diluted with blocking solution were added and incubated at room temperature for 2 hours. After washing three times with PBS, the cells were sealed with anti-fading agent (manufactured by DAKO, code number: S3023) and observed under a microscope. Note that the cell nuclei were visualized by contrast staining with DAPI solution (1 µg/mL, manufactured by Dojindo, product code: D523). In addition, fluorescence images were acquired with an AxioImager M1 microscope (manufactured by Carl Zeiss).

In immunostaining for the above cultured cells, the following antibodies were used to detect the antigen. Primary Antibodies:
Anti-Nestin mouse monoclonal antibody (manufactured by BD, clone Rat401, diluted to 1:200 for use),
Anti-Nanog rabbit polyclonal antibody (manufactured by Wako pure chemical, code number 018-27521, diluted to 1:200 for use),
Anti-Sox2 rabbit polyclonal antibody (manufactured by StemCell Technology, diluted to 1:500 for use), Anti-Oct4 mouse monoclonal antibody (manufactured by Cell Signaling Tech, diluted to 1:200 for use), Anti-GFP rat monoclonal antibody (manufactured by Nacalai Tesque, diluted to 1:500 for use),
Anti-Ki67 rabbit monoclonal antibody (manufactured by Thermo Fisher Scientific, diluted to 1:100 for use), and
Anti-active Caspase3 rabbit polyclonal antibody (manufactured by StemCell Technology, diluted to 1:1000 for use) .

Secondary Antibodies:
Alexa 488-conjugated anti-mouse IgG donkey polyclonal antibody (manufactured by Thermo Fisher Scientific, catalog number: R37120, diluted to 1:500 for use),
Alexa 488-conjugated anti-rabbit IgG donkey polyclonal antibody (manufactured by Thermo Fisher Scientific, catalog number: A-11008, diluted to 1:500 for use),
Alexa 594-conjugated anti-rabbit IgG donkey polyclonal antibody (manufactured by Thermo Fisher Scientific, catalog number: R37119, diluted to 1:500 for use),
Alexa 594-conjugated anti-mouse IgG donkey polyclonal antibody (manufactured by Thermo Fisher Scientific, catalog number: R37115, diluted to 1:500 for use), and
Alexa 488-conjugated anti-rat IgG goat antibody (manufactured by Jackson ImmunoResearch, diluted to 1:500 for use).

For tumors, 10-µm-thick sections were prepared and immunostained using the methods described by Kondo T. and Raff M., EMBO J. 2000; 19(9): 1998-2007, and Takanaga H, et al., Stem Cells. 2009; 27(1): 165-174. The following antibodies were used for this immunostaining.

Primary Antibodies:
Anti-Oct4 mouse monoclonal antibody (manufactured by Cell Signaling Tech, diluted to 1:200 for use)
Anti-Nanog rabbit polyclonal antibody (manufactured by Wako Pure Chemical, diluted to 1:200 for use)
Anti-Ki67 rabbit monoclonal antibody (manufactured by Thermo Fisher Scientific, diluted to 1:100 for use)
Anti-βIII tubulin mouse monoclonal antibody (manufactured by Sigma, diluted to 1:400 for use)
Anti-smooth muscle actin rabbit polyclonal antibody (manufactured by Proteintech, SMA, diluted to 1:200 for use)
Anti-a fetoprotein rabbit polyclonal antibody (manufactured by Proteintech, AFP, diluted to 1:100 for use) .

Secondary Antibodies:
Alexa 594-conjugated anti-mouse IgG donkey polyclonal antibody (manufactured by Thermo Fisher Scientific, catalog number: R37115, diluted to 1:500 for use)
Alexa 488-conjugated anti-rabbit IgG goat antibody (manufactured by Jackson ImmunoResearch, diluted to 1:500 for use).

### (Mixed Culture)

Mouse NSCs and mouse ES cells or mouse iPS cells were seeded in 2000 cells each, and cultured in ESC culture solution in the presence of 1 µM or 10 µM BRQ for 3 days. The cells were then analyzed by immunostaining as described above.

### (Quantitative RT-PCR)

Mouse ES cells and mouse iPS cells were cultured in 100 mm culture dishes coated with the above-described bovine epidermis-derived gelatin and ESC culture solution in the presence or absence of BRQ, and then the gene expression levels of Sox2, Oct4, and Nanog in these cells were measured by the following method.

First, total RNA was prepared from cells using the RNeasy kit (manufactured by Qiagen), and cDNA was synthesized using the Transcriptor First Strand cDNA Synthesis Kit (manufactured by Roche Applied Science).

Then, the cDNA was used as a template, and Thunderbird Cyber qPCR Mix (manufactured by TOYOBO) and Step One Plus (manufactured by Thermo Fisher Scientific) were used to perform real-time PCR. The reaction conditions were set at 95°C for 15 seconds and 60°C for 30 seconds for 40 cycles, and the oligonucleotide primers shown below were used to amplify each target gene.

Sox2 Gene:
Forward primer 5'-TGAAGAAGGATAAGTACACGCT-3' (SEQ ID NO: 1),
Reverse primer 5'-TCCTGCATCATGCTGTAGCTG-3' (SEQ ID NO: 2),
oct4 Gene:
   Forward primer 5'-CTGAAGCAGAAGAGGATCACC-3' (SEQ ID NO: 3)
   Reverse primer 5'-CCGCAGCTTACACATGTTCTT-3' (SEQ ID NO: 4),
nanog Gene:
   Forward primer 5'-CTGATTCTTCTACCAGTCCCAA-3' (SEQ ID NO: 5),
   Reverse primer 5'-AGAGTTCTTGCATCTGCTGGA-3' (SEQ ID NO: 6)
18S Ribosomal RNA Gene:
   Forward primer 5'-CGGACAGGATTGACAGATTG-3' (SEQ ID NO: 7),
   Reverse primer 5'-CAAATCGCTCCACCAACTAA-3' (SEQ ID NO: 8) .

Note that the expression level of each target gene detected in this way was normalized by the relative quantification method (delta delta Ct method) based on the expression level of 18S ribosomal RNA.

### (Vectors)

Vectors were constructed as described by Nishide K, et al., PLoS ONE. 2009; 4(8): e6869. and Takanaga H, et al., Stem Cells. 2009; 27(1): 165-174.

Specifically, full-length cDNA of mouse DHODH was amplified from mouse NSC-derived cDNA using KOD Plus-Ver. 2 polymerase (manufactured by TOYOBO Co., Ltd.) according to the use instruction manual. For this amplification, the 5'-primer (5'-AGAATTCAATGGGGGAACA-3' (SEQ ID NO: 9)) and the 3'-primer (5'-TTGGATTCTCTGCGGTC-3' (SEQ ID NO: 10)) were used. The amplified cDNA was inserted into the p3xFLAG CMV10 vector to prepare p3xFLAG CMV10-mDHODH.

In order to knockdown mouse DHODH, three short hairpin (sh) sequences targeting the gene were selected using InvivoGen's siRN Wizard software (http://www.sirnawizard.com/). These sh sequences were inserted into the psiRNA-h7SKhygro G1 expression vector (manufactured by InvivoGen), and psiRNA-h7SKhygro-mDHODHsh1-3 was prepared. The knockdown efficiency by these vectors was analyzed by Western blotting (see Fig. 14). As a result, DHODH sh1 and sh3, which showed high effects, were used for the knockdown experiments.

The target sequences of sh1 and sh3 for mouse DHODH are 5'-GGCTAGCTGTCtCTCTCT-3' (SEQ ID NO: 11) and 5'-GGAAGCTGTGTCTCTCtA-3' (SEQ ID NO: 12), respectively. The target sequence of sh (egfp) used as control is 5'-GCAAGCTGACCCGTGTTCA-3' (SEQ ID NO: 13).

The sequences of the cloned cDNAs were confirmed using BigDye Terminator Kit version 3.1 (manufactured by Applied Biosystems) and ABI Sequencer Model 3130xl (manufactured by Applied Biosystems).

Further, the vectors were also introduced into the cells using Lipofectamine 3000 (manufactured by Thermo Fisher Scientific) according to the use instruction manual.

### (Teratoma formation)

Pluripotent stem cells were cultured for 2 days in the presence or absence of BRQ. The viable cells (1 x 10⁶) were suspended in 50 µl of Matrigel (manufactured by BD Biosciences) and injected subcutaneously into the buttocks of 5-8-week-old NOD/SCID mice anesthetized with 10% pentobarbital. Four weeks after the injection, the mice were sacrificed to death, and the tumors were removed and photographed for their size measurement according to the method described in Tsukamoto Y, et al., Stem Cells. 2016; 34(8): 2016-2025. Based on the measurement values obtained, the volume of the tumors was calculated by the following formula.

Volume of tumor (cm³)=major diameter×minor diameter×minor diameter×1/2.

To investigate the suppression activity of BRQ on teratoma formation, 200 µl of saline or 200 µl of 25 mg/kg brequinar sodium salt (BRQ, manufactured by TOCRIS) PBS solution was administered daily intraperitoneally to mice bearing subcutaneous tumors. Five days after intraperitoneal administration, the mice were sacrificed to death, and the tumors were removed and photographed for pathological analysis according to the method described in Tsukamoto Y, et al., Stem Cells. 2016; 34(8): 2016-2025.

### (Western Blotting)

Western blotting was performed according to the method described in Takanaga H, et al., Stem Cells. 2009; 27(1): 165-174. For Western blotting and immunoprecipitation, the primary antibodies used were anti-FLAG M2 mouse antibody (manufactured by SIGMA, 10u/ml) and anti-GAPDH antibody (manufactured by Proteintech, diluted to 1:5000 for use), and the secondary antibody used was horseradish peroxidase-conjugated anti-mouse IgG antibody (manufactured by Santa Cruz, diluted to 1:5000 for use).

### (Statistical Analysis)

Comparisons between the two groups in terms of cell viability, gene expression level, and the like obtained above were analyzed by student t-test. Also, the Kaplan-Meier curve was used to estimate unadjusted time-to-event variables. P-values less than 0.05 (two-tailed) were considered to be significant.

### (Example 1) Study on Cytotoxic Activity of DHODH Inhibitors against Pluripotent Stem Cells

Mouse ES cells and mouse iPS cells were cultured for 3 days each in the presence of DHODH inhibitors (BRQ, leflunomide, teriflunomide, or vidofludimus), and viability was examined by MTT assay.

The results showed that all four DHODH inhibitors tested had cytotoxic activity against both types of pluripotent stem cells, as shown in Figs. 1 and 2. In particular, BRQ showed significant cytotoxic activity against pluripotent stem cells even at low concentrations (from 10 µM). Note that the IC₅₀ of BRQ, leflunomide, teriflunomide, and vidofludimus against human-derived DHODH is 6 to 20 nM, 98 µM, 1 µM, and 134 nM, respectively. Therefore, the difference in cytotoxic activity against pluripotent stem cells among DHODH inhibitors appears to be due to the difference in inhibitory activity.

### (Example 2) Study on Cytotoxic Activity of DHODH Inhibitors against Somatic Stem Cells and the Like

Mouse normal neural stem cells (mNSC), mNSCs cultured in the presence of 5% fetal bovine serum (mNSC+5%FCS), and mouse astrocytes were cultured in the presence of 10 µM BRQ for 3 days, and the viability was examined by MTT assay. As a result, it was found that neural stem cells and neurons (differentiated cells) were hyposensitive to BRQ, as shown in Fig. 3.

Further, other pluripotent stem cells (NT2: human embryonal carcinoma (EC) cells) and other somatic stem cells (PA6: mouse bone marrow-derived stromal cells, C2C12: mouse myoblasts) were also cultured in the presence of 10 µM BRQ for 3 days, and the viability was examined by MTT assay. As a result, BRQ showed significant cytotoxic activity in pluripotent stem cells (ES cells, iPS cells, and EC cells), as shown in Fig. 4. On the other hand, there was a statistically significant difference between the viability of ES cells and iPS cells and that of PA6 cells, C2C12 cells, neural stem cells, and astrocytes (all p<0.01). In other words, there was no significant cytotoxic activity of BRQ on somatic stem cells and somatic cells.

### (Example 3) Verification of Cell-Specific Damaging Activity of DHODH Inhibitors on Pluripotent Stem Cells

Pluripotent stem cells (mouse ES cells or mouse iPS cells) and somatic stem cells (mouse neural stem cells), which were assumed to be cells obtained by differentiation from the cells, were mixed and cultured in ES cell medium containing BRQ at various concentrations for 3 days, and immunostaining for Nestin, an NSC marker, and Nanog, a pluripotent stem cell marker, was performed. As a result, as shown in Figs. 5 and 6, ES cells and iPS cells were lost in the presence of 10 µM BRQ in the same manner as in Figs. 1 to 4 above, but no obvious cytotoxicity to neural stem cells was observed.

### (Example 4) Verification of Nucleoside Neutralizing Activity of DHODH Inhibitors against Cytotoxicity

Pyrimidine synthesis is regulated by the de novo (de novo synthesis) pathway and salvage pathway. DHODH is a key factor in de novo synthesis, catalyzing the fourth chemical reaction in the synthetic pathway. In view of this, the present inventor investigated whether the effect of eliminating pluripotent stem cells by DHODH inhibitors could be eliminated by adding nucleosides such as uridine, which is the starting material of the salvage pathway. More specifically, the present inventor verified whether the addition of a nucleoside (adenosine, guanosine, cytidine, or uridine) to the culture medium in the presence of BRQ could neutralize the cytotoxic activity of the inhibitors on pluripotent stem cells. As a result, as shown in Figs. 7 and 8, pyrimidine nucleosides (uridine and cytidine) showed neutralizing activity against the cytotoxic activity of BRQ, and uridine in particular showed strong neutralizing activity.

In addition, UMP, a product of the pyrimidine synthesis pathway, is converted to UDP and UTP. Furthermore, uridine diphosphate-N-acetylglucosamine (UDP-GlcNac) is also biosynthesized using UTP as a substrate. UDP-GlcNac is a substrate for O-linked N-acetylglucosaminyltransferase (OGT), which has been shown to be extensively involved in intracellular signal transduction. Therefore, the present inventor verified whether the cytotoxic activity of DHODH inhibitors on pluripotent stem cells could also be neutralized by the products from these UMPs. The results showed that UDP and UDP-GlcNac also neutralized the cytotoxic activity of BRQ, as shown in Figs. 9 and 10.

The present inventor also evaluated the neutralizing activity of nucleotide diphosphates (UDP, CDP, ADP, and GDP), which are intermediate substrates for nucleotide synthesis, against the cytotoxic activity of BRQ. As a result, strong neutralizing activity was observed for UDP (see Fig. 11).

The above results suggest that DHODH inhibitors, such as BRQ, exert their cytotoxic activity against pluripotent stem cells by inhibiting the pyrimidine synthesis pathway. The results also suggest that this cytotoxic activity can be suppressed by activating the salvage pathway.

### (Example 5) Verification of BRQ-Induced Cell Cycle Arrest and Cell Death in Pluripotent Stem Cells

In order to clarify what phenomenon BRQ, a DHODH inhibitor, causes on pluripotent stem cells, pluripotent stem cells were cultured in the presence or absence of BRQ for 2 days, and then bromo-deoxyuridine (BrdU) uptake assay and Casp3 immunostaining were performed to analyze their cell proliferation and cell death. The results showed that BRQ reduced the proliferation of pluripotent stem cells and strongly activated CASP3, as shown in Figs. 12 and 13.

In addition, to evaluate whether the BRQ-dependent cytotoxic activity was completely dependent on the inhibition of DHODH activity, two types of DHODH-specific shRNAs (see Fig. 14) were used to knockdown DHODH. As a result, the proliferation of pluripotent stem cells was reduced and CASP3 was strongly activated, as shown in Figs. 15 to 18. Thus, the same phenomenon as in BRQ-treated cells was also produced by DHODH knockdown, suggesting that the aforementioned BRQ-dependent cytotoxicity was exerted by inhibiting DHODH activity.

### (Example 6) Verification of Effects of DHODH Inhibitors on Marker Molecules of Pluripotent Stem Cells

It has been clarified that pluripotent stem cells express high levels of three types of transcription factors (Sox2, Oct4, and Nanog), which are involved in the promotion of self-renewal capability and maintenance of undifferentiated state. In view of this, the present inventor verified the effects of DHODH inhibitors on the marker molecules of these pluripotent stem cells.

Specifically, first, the expression of Sox2 in pluripotent stem cells (mouse iPS cells and mouse ES cells) and somatic stem cells (mouse neural stem cells) cultured in the presence of BRQ for 24 hours was detected by fluorescence immunostaining. As a result, as shown in Figs. 19 and 20, inhibition of cell proliferation or cell death was already observed in ES cells and iPS cells after 24 hours of culture in the presence of BRQ. In addition, the expression of Sox2 was not observed in about 30% of both types of pluripotent stem cells. On the other hand, no similar changes were observed in somatic stem cells.

Next, the gene expression levels of Sox2, Oct4, and Nanog in pluripotent stem cells (mouse iPS cells and mouse ES cells) cultured for 1 or 2 days in the presence of BRQ were measured by PCR. As a result, as shown in Fig. 21, it was found that the expression levels of the marker genes of pluripotent stem cells were generally decreased in the cells treated with BRQ for 2 days.

In addition, the expression of Nanog in pluripotent stem cells (mouse iPS cells and mouse ES cells) cultured for 2 days in the presence of BRQ was also detected by fluorescent immunostaining. As a result, as shown in Fig. 22, it was found that Nanog, which is normally localized in the nuclei of pluripotent stem cells, was distributed in the cytoplasm after BRQ treatment.

The above results suggest that DHODH inhibitors reduce the expression levels of the three types of transcription factors (Sox2, Oct4, and Nanog), which are involved in the promotion of self-renewal capability and maintenance of undifferentiated state, and that the change in subcellular localization can lead to cytotoxicity and even cell death in pluripotent stem cells.

### (Example 7) CRM1-Dependent Nuclear Export of Pluripotent Stem Cell Marker Molecules Induced by DHODH Inhibitors

Pluripotent stem cells were cultured in the presence or absence of BRQ or Z-VAD, a pan-caspase inhibitor. Two days after BRQ treatment, the numbers of Oct4-positive cells and Nanog-positive cells in pluripotent stem cells (ES cells and iPS cells) were measured.

As a result, as shown in Fig. 23, the percentages of Oct4-positive cells and Nanog-positive cells in the BRQ-treated pluripotent stem cells were significantly decreased compared to those in the DMSO-treated control pluripotent stem cells (In ES cells treated with BRQ, Oct4-positive cells: 2%, Nanog-positive cells: 6%. In iPS cells treated with BRQ, Oct4-positive cells: 0%, Nanog-positive cells: 0%. In ES cells treated with DMSO, Oct4-positive cells: 85%, Nanog-positive cells: 86%. In iPS cells treated with DMSO, Oct4-positive cells: 94%, Nanog-positive cells: 93%).

Also, the addition of Z-VAD caused a slight delay in BRQ-dependent cell death, but did not restore the aforementioned decrease in the percentages of Nanog-positive cells and Oct4-positive cells in pluripotent stem cells treated with BRQ (In ES cells treated with BRQ and Z-VAD, Oct4-positive cells: 5%, Nanog-positive cells: 2%. In iPS cells treated with BRQ and Z-VAD, Oct4-positive cells: 3%, Nanog-positive cells: 5%) (see Fig. 23).

In addition, pluripotent stem cells were cultured in medium supplemented with leptomycin B (LMB) in the presence or absence of BRQ, and the nuclear localization of Nanog and Oct4 was analyzed. LMB is a specific inhibitor of CRM1, also known as Exportin 1.

As a result, as shown in Fig. 24, LMB completely suppressed the nuclear export of Nanog and Oct4 in pluripotent stem cells treated with BRQ. This suggests that the elimination of Nanog and Oct4 from the nucleus by BRQ occurred in a CRM1-dependent manner.

### (Example 8) Verification of Suppressive Effect of DHODH Inhibitor on Tumorigenicity of Pluripotent Stem Cells

The present inventor investigated the tumorigenicity of pluripotent stem cells pretreated with BRQ, a DHODH inhibitor. Specifically, first, pluripotent stem cells were cultured for 2 days in medium supplemented with DMSO only or 10 µM BRQ, and then the viable ones were injected subcutaneously into the buttocks of NOD/SCID mice. Then, 4 weeks after transplantation, the tumors were removed and observed.

As a result, as shown in Figs. 25 and 26, the cells pretreated with BRQ did not proliferate. On the other hand, tumor formation was observed in the cells treated with DMSO.

In addition, to investigate the anti-teratoma activity of BRQ, pluripotent stem cells were first transplanted under the buttocks skin of NOD/SCID mice to form tumors that reached a size of over 100 mm³. Then, BRQ was injected intraperitoneally once every 3 days, and the tumors were removed from the mice on the third day after the fifth intraperitoneal injection was given.

As shown in Fig. 27, tumor proliferation was suppressed by BRQ administration. Although not shown in the figure, no visible side effects were observed in the BRQ-treated mice. Also, as shown in Fig. 28, the sizes of teratomas treated with DMSO and BRQ were 0.55 cm³ and 0.12 cm³ for ES cell-derived ones, and 1.37 cm³ and 0.32 cm³ for iPS cell-derived ones, respectively.

Next, sections of these tumors were prepared, immunolabeled for the proliferation marker Ki67, pluripotent stem cell markers (Oct4 and Nanog), and differentiation markers (ATF, βIII tubulin, and SMA), and observed.

As a result, as shown in Fig. 29, the Ki67-positive proliferating cells in BRQ-treated tumors were significantly reduced compared to those in DMSO-treated tumors. In addition, as shown in Fig. 30, the number of Oct4-positive cells and Nanog-positive cells in BRQ-treated tumors was significantly decreased, while many cells expressing pluripotent stem cell markers were found in DMSO-treated tumors. On the other hand, cells expressing each of the markers, ATF, βIII tubulin, and SMA, in the three germ layers were similarly contained in both tumors.

Therefore, it has become clear that DHODH inhibitors can suppress tumor formation by specifically eliminating undifferentiated pluripotent stem cells without causing obvious cytotoxicity to differentiated cells and mice.

### [Industrial Applicability]

As described above, according to the present invention, it is possible to eliminate undifferentiated pluripotent stem cells without causing significant cytotoxicity to the differentiated cells. Therefore, the present invention is excellent in eliminating undifferentiated pluripotent stem cells remaining in a cell group induced to differentiate from pluripotent stem cells, and is thus useful in regenerative medicine and the like, where the risk of tumorigenesis is reduced and there are fewer side effects.

## Claims

1. A composition for eliminating undifferentiated pluripotent stem cells remaining in a cell group induced to differentiate from pluripotent stem cells, comprising: a dihydroorotate dehydrogenase inhibitor as an active ingredient.

2. The composition according to claim 1, wherein the pluripotent stem cells are at least one pluripotent stem cell selected from the group consisting of embryonic stem cells (ES cells), induced pluripotent stem cells (iPS cells), and embryonal carcinoma cells (EC cells).

3. The composition according to claim 1 or 2, wherein the dihydroorotate dehydrogenase inhibitor is brequinar.

4. The composition according to any one of claims 1 to 3, wherein the cell group is a cell group containing somatic stem cells induced to differentiate from the pluripotent stem cells.

5. A method for eliminating undifferentiated pluripotent stem cells remaining in a cell group, comprising: contacting a cell group induced to differentiate from pluripotent stem cells with a dihydroorotate dehydrogenase inhibitor.

6. The method according to claim 5, wherein the pluripotent stem cells are at least one pluripotent stem cell selected from the group consisting of embryonic stem cells (ES cells), induced pluripotent stem cells (iPS cells), and embryonal carcinoma cells (EC cells).

7. The method according to claim 5 or 6, wherein the dihydroorotate dehydrogenase inhibitor is brequinar.

8. The method according to any one of claims 5 to 7, wherein the cell group is a cell group containing somatic stem cells induced to differentiate from the pluripotent stem cells.
